(19) **Europäisches Patentamt / European Patent Office / Office européen des brevets**

(11) **EP 4 722 247 A1**

(12) **EUROPEAN PATENT APPLICATION**
published in accordance with Art. 153(4) EPC

(43) Date of publication:
08.04.2026 Bulletin 2026/15

(21) Application number: 24814635.9

(22) Date of filing: 03.06.2024

(51) International Patent Classification (IPC):
C07K 16/28 $^{(2006.01)}$  C12N 15/13 $^{(2006.01)}$
A61K 39/395 $^{(2006.01)}$  A61P 35/00 $^{(2006.01)}$

(52) Cooperative Patent Classification (CPC):
A61K 39/395; A61P 35/00; C07K 16/00;
C07K 16/28

(86) International application number:
PCT/CN2024/096977

(87) International publication number:
WO 2024/245441 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 02.06.2023 CN 202310652534

(71) Applicant: Oricell Therapeutics Co., Ltd.
Shanghai 201203 (CN)

(72) Inventors:
• LI, Xiaopei
  Shanghai 201203 (CN)
• HE, Xiaowen
  Shanghai 201203 (CN)
• DENG, Qi
  Shanghai 201203 (CN)
• LI, Xianyang
  Shanghai 201203 (CN)
• WANG, Huajing
  Shanghai 201203 (CN)
• ZHANG, Yuchen
  Shanghai 201203 (CN)

(74) Representative: Weickmann & Weickmann
PartmbB
Postfach 860 820
81635 München (DE)

Remarks:
The complete document including Reference
Table(s) and the Sequence Listing(s) can be
downloaded from the EPO website

(54) **ANTIGEN BINDING PROTEIN TARGETING CD39 AND USE THEREOF**

(57) An antibody or an antigen-binding fragment thereof, which can specifically bind to a CD39 protein. Further provided is the use of the antibody or the antigen-binding fragment thereof in the preparation of a drug for preventing and/or treating diseases.

EP 4 722 247 A1

**Description**

**Field of the Invention**

The present application relates to the field of biomedicine, and particularly to an antibody targeting CD39 or an antigen-binding fragment thereof, and a use thereof.

**Background of the Invention**

CD39, also known as NTPDase1, is an enzyme on the cell membrane and belongs to the E-NTPDase family (extracellular nucleotide diphosphatase family). It is mainly responsible for the hydrolysis of extracellular ATP and ADP, converting them into AMP, which is subsequently degraded into adenosine by a downstream CD73 molecule. The main function of CD39 is to regulate the level of extracellular nucleotides and participate in various physiological and pathological processes.

In the immune system, CD39 plays an important role in regulating and controlling immune responses. CD39 reduces the concentration of extracellular ATP and ADP in inflammatory and immune responses by degrading them. This process can reduce the stimulatory effects of extracellular ATP and ADP on immune cells, thereby alleviating inflammatory responses and immune overactivation. In addition, adenosine produced by the ATP-CD39-CD73-adenosine pathway can also inhibit the activity of various immune cells by binding to adenosine receptors, further suppressing immune responses.

CD39 plays an important role in the development and progression of various diseases. Inflammatory diseases (such as rheumatoid arthritis, inflammatory bowel disease) and immune diseases (such as autoimmune diseases, organ transplant rejection) are all associated with the abnormal activation of the immune system, and the dysfunction of CD39 may be a key factor among them.

In the tumor microenvironment, there are multiple immunosuppressive mechanisms, including an increase in immunosuppressive cells, the production of immunosuppressive factors, and an increase in the expression of immunosuppressive receptors. The function of immune cells is suppressed, leading to a weakened anti-tumor immune response. Therefore, researchers have been constantly seeking new immunological targets for tumors in order to develop novel immunotherapeutic strategies.

Studies have shown that the expression level of CD39 is often markedly increased in tumor tissues and is closely related to the progression and poor prognosis of tumors. CD39 plays an important role in tumor immune escape and drug resistance. The hypoxic microenvironment of tumors usually leads to extensive cell death, thereby releasing a large amount of ATP. The widely upregulated CD39 in the tumor microenvironment degrades extracellular ATP and ADP to produce adenosine, thereby reducing the pro-inflammatory effects of ATP and ADP on the one hand, and increasing the concentration of adenosine on the other hand to further suppress the activity and function of immune cells. In the tumor microenvironment, CD39 is often markedly upregulated in various cells, which is also related to the immune escape of tumors and the resistance to immunotherapy. Therefore, CD39, as an enzyme that plays a crucial role in immune regulation, is a potential therapeutic target for diseases. The development of corresponding antibodies or inhibitors has extensive application value.

**Summary of the Invention**

The present application provides an antibody or an antigen-binding fragment thereof, which can specifically bind to a CD39 protein. The antibody or the antigen-binding fragment thereof has one or more of the following properties: (1) capable of binding to CD39 at a KD value of $1 \times 10^{-8}$ M or below; (2) capable of binding to human CD39, mouse CD39, or cynomolgus monkey CD39; (3) capable of effectively inhibiting the enzymatic activity of the CD39 protein; (4) capable of improving the function of immune cells, for example, promoting the proliferation of the immune cells; (5) capable of reducing the immunosuppressive function of regulatory T cells and reactivating exhaustion T cells.

In one aspect, the present application provides an antibody or an antigen-binding fragment thereof, which can specifically bind to a CD39 protein. The antibody or the antigen-binding fragment thereof includes at least one CDR in an antibody heavy chain variable region (VH), wherein the VH includes an amino acid sequence as set forth in any one of SEQ ID NO: 8, SEQ ID NO: 16, SEQ ID NO: 21, SEQ ID NO: 27, SEQ ID NO: 32, SEQ ID NO: 37, and SEQ ID NO: 41.

In some embodiments, the antibody or the antigen-binding fragment thereof includes an HCDR3, wherein the HCDR3 includes an amino acid sequence as set forth in any one of SEQ ID NO: 3, SEQ ID NO: 54, and SEQ ID NO: 55.

In some embodiments, the antibody or the antigen-binding fragment thereof includes an HCDR3, wherein the HCDR3 includes an amino acid sequence as set forth in any one of SEQ ID NO: 3, SEQ ID NO: 11, SEQ ID NO: 18, SEQ ID NO: 24, SEQ ID NO: 29, and SEQ ID NO: 33.

In some embodiments, the antibody or the antigen-binding fragment thereof includes an HCDR2, wherein the HCDR2 includes an amino acid sequence as set forth in any one of SEQ ID NO: 2, SEQ ID NO: 10, SEQ ID NO: 23, and SEQ ID NO: 28.

In some embodiments, the antibody or the antigen-binding fragment thereof includes an HCDR1, wherein the HCDR1 includes an amino acid sequence as set forth in any one of SEQ ID NO: 1, SEQ ID NO: 53, and SEQ ID NO: 22.

In some embodiments, the antibody or the antigen-binding fragment thereof includes an HCDR1, wherein the HCDR1 includes an amino acid sequence as set forth in any one of SEQ ID NO: 1, SEQ ID NO: 9, SEQ ID NO: 17, and SEQ ID NO: 22.

In some embodiments, the antibody or the antigen-binding fragment thereof includes an HCDR1, an HCDR2, and an HCDR3, wherein the HCDR1, HCDR2, and HCDR3 includes amino acid sequences selected from a group consisting of:

(1) the HCDR1 includes an amino acid sequence as set forth in SEQ ID NO: 1, the HCDR2 includes an amino acid sequence as set forth in SEQ ID NO: 2, and the HCDR3 includes an amino acid sequence as set forth in SEQ ID NO: 3;
(2) the HCDR1 includes an amino acid sequence as set forth in SEQ ID NO: 9, the HCDR2 includes an amino acid sequence as set forth in SEQ ID NO: 10, and the HCDR3 includes an amino acid sequence as set forth in SEQ ID NO: 11;
(3) the HCDR1 includes an amino acid sequence as set forth in SEQ ID NO: 17, the HCDR2 includes an amino acid sequence as set forth in SEQ ID NO: 10, and the HCDR3 includes an amino acid sequence as set forth in SEQ ID NO: 18;
(4) the HCDR1 includes an amino acid sequence as set forth in SEQ ID NO: 22, the HCDR2 includes an amino acid sequence as set forth in SEQ ID NO: 23, and the HCDR3 includes an amino acid sequence as set forth in SEQ ID NO: 24;
(5) the HCDR1 includes an amino acid sequence as set forth in SEQ ID NO: 22, the HCDR2 includes an amino acid sequence as set forth in SEQ ID NO: 28, and the HCDR3 includes an amino acid sequence as set forth in SEQ ID NO: 29; or
(6) the HCDR1 includes an amino acid sequence as set forth in SEQ ID NO: 22, the HCDR2 includes an amino acid sequence as set forth in SEQ ID NO: 28, and the HCDR3 includes an amino acid sequence as set forth in SEQ ID NO: 33.

In some embodiments, the antibody or the antigen-binding fragment thereof includes an H-FR1, wherein a C-terminus of the H-FR1 is linked to an N-terminus of the HCDR1 directly or indirectly, and the H-FR1 includes an amino acid sequence as set forth in SEQ ID NO: 56.

In some embodiments, the H-FR1 of the antibody or the antigen-binding fragment thereof includes an amino acid sequence as set forth in any one of SEQ ID NO: 4, SEQ ID NO: 12, SEQ ID NO: 19, SEQ ID NO: 34, and SEQ ID NO: 38.

In some embodiments, the antibody or the antigen-binding fragment thereof includes an H-FR2, wherein the H-FR2 is located between the HCDR1 and the HCDR2, and the H-FR2 includes an amino acid sequence as set forth in SEQ ID NO: 57.

In some embodiments, the H-FR2 of the antibody or the antigen-binding fragment thereof includes an amino acid sequence as set forth in any one of SEQ ID NO: 5, SEQ ID NO: 13, SEQ ID NO: 20, SEQ ID NO: 25, SEQ ID NO: 30, and SEQ ID NO: 35.

In some embodiments, the antibody or the antigen-binding fragment thereof includes an H-FR3, wherein the H-FR3 is located between the HCDR2 and the HCDR3, and the H-FR3 includes an amino acid sequence as set forth in SEQ ID NO: 58.

In some embodiments, the H-FR3 of the antibody or the antigen-binding fragment thereof includes an amino acid sequence as set forth in any one of SEQ ID NO: 6, SEQ ID NO: 14, SEQ ID NO: 26, SEQ ID NO: 31, SEQ ID NO: 36, and SEQ ID NO: 40.

In some embodiments, the antibody or the antigen-binding fragment thereof includes an H-FR4, wherein an N-terminus of the H-FR4 is linked to a C-terminus of the HCDR3, and the H-FR4 includes an amino acid sequence as set forth in SEQ ID NO: 59.

In some embodiments, the H-FR4 of the antibody or the antigen-binding fragment thereof includes an amino acid sequence as set forth in any one of SEQ ID NO: 7, SEQ ID NO: 15, and SEQ ID NO: 39.

In some embodiments, the antibody or the antigen-binding fragment thereof includes a VH, wherein the VH includes an amino acid sequence as set forth in any one of SEQ ID NO: 8, SEQ ID NO: 16, SEQ ID NO: 21, SEQ ID NO: 27, SEQ ID NO: 32, SEQ ID NO: 37, and SEQ ID NO: 41.

In some embodiments, the antigen-binding fragment is a VHH.

In some embodiments, the VHH includes an amino acid sequence as set forth in any one of SEQ ID NO: 8, SEQ ID NO: 16, SEQ ID NO: 21, SEQ ID NO: 27, SEQ ID NO: 32, SEQ ID NO: 37, and SEQ ID NO: 41.

In some embodiments, the antibody or the antigen-binding fragment thereof includes an antibody heavy chain constant region.

In some embodiments, the antibody heavy chain constant region is derived from a human IgG constant region.

In some embodiments, the antibody heavy chain constant region is derived from a human IgG4 constant region.

In some embodiments, the antibody heavy chain constant region includes an amino acid sequence as set forth in SEQ ID NO: 44.

In some embodiments, the antigen-binding fragment includes Fab, Fab', an Fv fragment, F(ab')$_2$, F(ab)$_2$, scFv, di-scFv and/or dAb.

In some embodiments, the antibody is selected from one or more of a group consisting of: a monoclonal antibody, a chimeric antibody, a humanized antibody, and a fully human antibody.

In some embodiments, the CD39 protein is human CD39 protein, mouse CD39 protein or cynomolgus monkey CD39 protein.

In another aspect, the present application also provides a bispecific antibody or an antigen-binding fragment thereof, which includes a first binding domain that specifically binds to a CD39 protein, wherein the first binding domain includes the antibody or the antigen-binding fragment thereof.

In some embodiments, the first binding domain that specifically binds to a CD39 protein is a VHH that specifically binds to CD39.

In some embodiments, the bispecific antibody or the antigen-binding fragment thereof includes a second binding domain that specifically binds to a PD-L1 protein.

In some embodiments, the second binding domain that specifically binds to a PD-L1 protein includes a PD-L1 antibody or an antigen-binding fragment thereof.

In some embodiments, the PD-L1 antibody or the antigen-binding fragment thereof includes an HCDR1, an HCDR2, and an HCDR3, wherein the HCDR1 includes an amino acid sequence as set forth in SEQ ID NO: 45, the HCDR2 includes an amino acid sequence as set forth in SEQ ID NO: 46, and the HCDR3 includes an amino acid sequence as set forth in SEQ ID NO: 47.

In some embodiments, the PD-L1 antibody or the antigen-binding fragment thereof includes a VH, wherein the VH includes an amino acid sequence as set forth in SEQ ID NO: 48.

In some embodiments, the PD-L1 antibody or the antigen-binding fragment thereof includes an LCDR1, an LCDR2, and an LCDR3, wherein the LCDR1 includes an amino acid sequence as set forth in SEQ ID NO: 49, the LCDR2 includes an amino acid sequence as set forth in SEQ ID NO: 50(GNS), and the LCDR3 includes an amino acid sequence as set forth in SEQ ID NO: 51.

In some embodiments, the PD-L1 antibody or the antigen-binding fragment thereof includes a VL, wherein the VL includes an amino acid sequence as set forth in SEQ ID NO: 52.

In some embodiments, the PD-L1 antibody or the antigen-binding fragment thereof includes an antibody heavy chain constant region.

In some embodiments, the PD-L1 antibody or the antigen-binding fragment thereof includes an antibody light chain constant region.

In some embodiments, the antibody heavy chain constant region is derived from a human IgG constant region.

In some embodiments, a C-terminus of the VHH that specifically binds to CD39 in the bispecific antibody or the antigen-binding fragment thereof is linked to an N-terminus of the PD-L1 antibody or the antigen-binding fragment thereof directly or indirectly.

In some embodiments, an N-terminus of the VHH that specifically binds to CD39 in the bispecific antibody or the antigen-binding fragment thereof is linked to a C-terminus of the PD-L1 antibody or the antigen-binding fragment thereof directly or indirectly.

In some embodiments, the indirect linkage includes linkage through a linker.

In some embodiments, the linker includes an amino acid sequence as set forth in (G4S)n, wherein n is a positive integer from 1 to 10.

In some embodiments, the bispecific antibody or the antigen-binding fragment thereof includes a first polypeptide chain and a second polypeptide chain, wherein the first polypeptide chain includes a heavy chain variable region of the PD-L1 antibody or the antigen-binding fragment thereof, a heavy chain constant region of the PD-L1 antibody or the antigen-binding fragment thereof, and the VHH that specifically binds to CD39, and the second polypeptide chain includes a light chain variable region and a light chain constant region of the PD-L1 antibody or the antigen-binding fragment thereof.

In some embodiments, the bispecific antibody or the antigen-binding fragment thereof includes a first polypeptide chain and a second polypeptide chain, wherein the first polypeptide chain includes a heavy chain variable region of the PD-L1 antibody or the antigen-binding fragment thereof, a heavy chain constant region of the PD-L1 antibody or the antigen-binding fragment thereof, a linker and the VHH that specifically binds to CD39, and the second polypeptide chain includes a light chain variable region and a light chain constant region of the PD-L1 antibody or the antigen-binding fragment thereof.

In some embodiments, the bispecific antibody or the antigen-binding fragment thereof includes a first polypeptide chain and a second polypeptide chain, wherein the first polypeptide chain includes, sequentially from N-terminus to C-terminus: a heavy chain variable region of the PD-L1 antibody or the antigen-binding fragment thereof, a heavy chain constant region of the PD-L1 antibody or the antigen-binding fragment thereof, a linker and the VHH that specifically binds to CD39, and the second polypeptide chain includes a light chain variable region and a light chain constant region of the PD-L1 antibody or the antigen-binding fragment thereof.

In some embodiments, the bispecific antibody or the antigen-binding fragment thereof includes a first polypeptide chain and a second polypeptide chain, wherein the first polypeptide chain includes, sequentially from N-terminus to C-terminus: the VHH that specifically binds to CD39, a linker, a heavy chain variable region of the PD-L1 antibody or the antigen-binding fragment thereof, and a heavy chain constant region of the PD-L1 antibody or the antigen-binding fragment thereof, and the second polypeptide chain includes a light chain variable region and a light chain constant region of the PD-L1 antibody or the antigen-binding fragment thereof.

In another aspect, the present application also provides a polypeptide, which includes the antibody or the antigen-binding fragment thereof, or the bispecific antibody or the antigen-binding fragment thereof.

In another aspect, the present application also provides one or more isolated nucleic acid molecules, which encode the antibody or the antigen-binding fragment thereof or the bispecific antibody or the antigen-binding fragment thereof.

In some embodiments, the nucleic acid molecule also includes nucleic acid molecule encoding other proteins.

In some embodiments, the other proteins include chimeric antigen receptors (CARs).

In another aspect, the present application also provides a vector, which includes the nucleic acid molecule.

In some embodiments, the nucleic acid molecule encoding the other proteins and the nucleic acid molecule encoding the antibody or the antigen-binding fragment thereof are located within the same vector.

In some embodiments, the nucleic acid molecule encoding the other proteins and the nucleic acid molecule encoding the antibody or the antigen-binding fragment thereof are located within different vectors.

In another aspect, the present application also provides a cell, which includes the nucleic acid molecule or the vector.

In some embodiments, the cell includes an immune cell.

In some embodiments, the immune cell includes a T cell, an NK cell, and/or a macrophage.

In some embodiments, the cell also includes and/or expresses other proteins.

In some embodiments, the other proteins also include chimeric antigen receptors (CARs).

In another aspect, the present application also provides a drug combination, which includes the following components:

(1) the antibody or the antigen-binding fragment thereof, or the bispecific antibody or the antigen-binding fragment thereof;
(2) a cell.

In some embodiments, the cell includes a modified cell or an unmodified cell.

In some embodiments, the modified cell includes and/or expresses a CAR.

In some embodiments, the cell includes an immune cell.

In some embodiments, the cell includes a T cell, an NK cell, and/or a macrophage.

In another aspect, the present application also provides a method for preparing the antibody or the antigen-binding fragment thereof or the bispecific antibody or the antigen-binding fragment thereof, which includes culturing the cell under conditions enabling the expression of the antibody or the antigen-binding fragment thereof or the bispecific antibody or the antigen-binding fragment thereof.

In another aspect, the present application also provides a use of the antibody or the antigen-binding fragment thereof, the bispecific antibody or the antigen-binding fragment thereof, the nucleic acid molecule, the cell, the drug combination, or the pharmaceutical composition in the preparation of a drug for preventing and/or treating a disease and/or a disorder.

In another aspect, the present application also provides a method for preventing and/or treating a disease and/or a disorder, which includes administering to a subject in need thereof the antibody or the antigen-binding fragment thereof, the bispecific antibody or the antigen-binding fragment thereof, the nucleic acid molecule, the cell, the drug combination, or the pharmaceutical composition.

In another aspect, the present application also provides the antibody or the antigen-binding fragment thereof, the bispecific antibody or the antigen-binding fragment thereof, the nucleic acid molecule, the cell, the drug combination, or the pharmaceutical composition, which are used for preventing and/or treating a disease and/or a disorder.

In some embodiments, the disease and/or disorder includes a tumor.

In some embodiments, the tumor includes a solid tumor and/or a hematological tumor.

In some embodiments, the tumor includes a tumor affected by CD39 activity.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

## Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:

Fig. 1 shows the affinity of the antibody or the antigen-binding fragment thereof as described in the present application to a human CD39 protein.

Figs. 2A-2B show the binding of the antibody or the antigen-binding fragment thereof as described in the present application to a human CD39 on the cell surface.

Figs. 3A-3C show the binding of the antibody or the antigen-binding fragment thereof as described in the present application to human, mouse, cynomolgus monkey CD39 proteins.

Figs. 4A-4B show the detection of enzymatic activity inhibition function at the protein level by the antibody or the antigen-binding fragment thereof as described in the present application.

Figs. 5A-5B show the detection of enzymatic activity inhibition function at the cellular level by the antibody or the antigen-binding fragment thereof as described in the present application.

Fig. 6 shows the proliferation-promoting effect of the antibody or the antigen-binding fragment thereof as described in the present application on PBMC-derived T cells.

Fig. 7 shows a simplified design diagram of the CAR structure of CAR-T cells that autocrinely secrete the antibody or the antigen-binding fragment thereof as described in the present application.

Fig. 8 shows the detection of the expression of inhibitory markers on the surface of CAR-T cells that autocrinely secrete the antibody or the antigen-binding fragment thereof as described in the present application after stimulation by tumor cells.

Fig. 9 shows the affinity of the humanized antibody or the antigen-binding fragment thereof as described in the present application to a human CD39 protein.

Fig. 10 shows the detection of enzymatic activity inhibition function at the protein level by the humanized antibody or the antigen-binding fragment thereof as described in the present application.

Fig. 11 shows the detection of enzymatic activity inhibition function at the cellular level by the humanized antibody or the antigen-binding fragment thereof as described in the present application.

Fig. 12 shows the improving effect of the humanized antibody or the antigen-binding fragment thereof as described in the present application on the proliferation of CAR-T cells.

Fig. 13 shows the structural design diagram of the bispecific antibody as described in the present application.

Fig. 14 shows the detection of the purity of the bispecific antibody as described in the present application.

Figs. 15A-15C show the tumor-suppressive effect of the bispecific antibody as described in the present application.

Fig. 16 shows the structural schematic diagrams of MSLN-CART that does not secrete antibodies or that autocrinely secretes B391-hF04, YN035(scFv), B391-hF04+YN035(scFv) antibodies.

Fig. 17A shows the statistics of tumor growth changes after the administration of different groups of CAR-T cells.

Fig. 17B shows the tumor volume changes after the administration of different groups of CAR-T cells.

Fig. 17C shows the body weight changes of mice after the administration of different groups of CAR-T cells.

## Detailed Description of the Embodiments

The implementation of the present application will be illustrated in the following specific examples, and other advantages and effects of the present application will be easily known by those familiar with this technology from the content disclosed in the specification.

## Definition of Terms

In the present application, the term "antibody" generally refers to a polypeptide molecule capable of specifically recognizing and/or neutralizing a specific antigen. For example, the antibody may include an immunoglobulin composed of a heavy (H) chain and a light (L) chain and include any molecules containing an antigen-binding moiety thereof. The term "antibody" includes a monoclonal antibody, an antibody fragment or an antibody derivative, including but not limited to a human antibody, a humanized antibody, a chimeric antibody, a single-chain antibody (e.g., scFv), a single-domain antibody (nanobody), and an antibody fragment binding to an antigen (e.g., Fab, Fab', (Fab)$_2$, and a VHH fragment). The term "antibody" also includes all recombinant forms of the antibody, such as antibodies expressed in prokaryotic cells, unglycosylated antibodies, as well as any antibody fragments binding to an antigen as described herein and derivatives thereof. Each chain may be composed of a variable region and a constant region. The variable region can be further differentiated into hypervariable regions called complementarity determining regions (CDRs), which are interspersed in more conserved regions called framework regions (FRs). Each of VH and/or VL can be composed of three CDRs and four FRs, which are arranged in the following order from the amino terminus to the carboxyl terminus: FR1, CDR1, FR2, CDR2, FR3, CDR3, and FR4. The variable regions of the heavy chain and the light chain contain binding domains interacting with antigens. The constant region of an antibody can mediate the binding of the immunoglobulin to the host tissue or factor, including various cells (e.g., effector cells) of the immune system and the first component of the classical complement system (C1q).

In the present application, the term "antigen-binding fragment" refers to a part of an intact antibody, specifically the antigen determining variable region of the intact antibody. For example, the antigen-binding fragment may include Fab, Fab', F(ab')2, an Fv fragment and a single-chain Fv fragment, a tandem Fv fragment, a VHH, a bispecific antibody. For example, the antigen-binding fragment may be a VHH. For example, the antigen-binding fragment can bind to CD39. For example, the antigen-binding fragment can be specific for CD39. In the present application, the term "VHH" generally refers to an antibody containing the variable antigen-binding domain of a heavy chain antibody. The VHH can also be referred to as a nanobody (Nb) and/or a single-domain antibody.

In the present application, the term "$K_D$" can be used interchangeably with "KD" and generally refers to the dissociation equilibrium constant for a specific antibody-antigen interaction, measured in M (mol/L). KD can be calculated from the concentrations of a substance AB and substances A and B obtained from the dissociation of the substance AB: KD =c (A) * c (B) /c (AB). From this formula, it can be known that the larger the KD value, the more dissociation there is, indicating a weaker affinity between substances A and B; conversely, the smaller the KD value, the less dissociation there is, indicating a stronger affinity between substances A and B.

In the present application, the term "monoclonal antibody" generally refers to a population of antibodies that are substantially homologous, meaning that all antibodies in this population are identical except for naturally occurring mutations that may exist in trace amounts. Monoclonal antibodies are highly specific and directly target a single antigenic site. In addition, unlike polyclonal antibody preparations that include different antibodies targeting different determinants (epitopes), the modifier "monoclonal" for each monoclonal antibody targeting a single determinant on the antigen is not interpreted as requiring the production of antibodies through any special methods. For example, monoclonal antibodies can be prepared by hybridoma technology or produced in bacterial, eukaryotic animal or plant cells by using recombinant DNA methods. Monoclonal antibodies can also be obtained from a phage antibody library, by using the technology as described in, e.g., Clackson et al., Nature, 352:624-628 (1991) and Marks et al., Mol. Biol., 222:581-597 (1991).

In the present application, the term "single-chain antibody" generally refers to (scFv), a molecule composed of the heavy chain variable regions and the light chain variable regions of an antibody linked by short peptide linkers.

In the present application, the term "chimeric antibody" generally refers to such an antibody in which a part of the amino acid sequence of each heavy chain or light chain is homologous to the corresponding amino acid sequence in an antibody derived from a specific species or belongs to a particular class, while the remaining segment of the chain is homologous to the corresponding sequence in another species. For example, the variable regions of the light chain or the heavy chain are derived from the variable region of the antibody of an animal species (e.g., mice, rats, and the like), while the constant part is homologous to the sequence of an antibody derived from another species (e.g., humans). For example, to obtain a chimeric antibody, the variable regions can be produced by using non-human B cells or hybridoma cells, while the constant region combined therewith is derived from humans. The variable regions have the advantage of being easy to prepare, and their specificity is not affected by the origin of the constant region combined therewith. Meanwhile, since the constant region of the chimeric antibody can be derived from humans, the chimeric antibody is less likely to elicit an immune response when being injected than an antibody of which the constant region is not derived from humans.

In the present application, the term "humanized antibody" generally refers to modified antibodies obtained by using genetic engineering techniques to reduce the immunogenicity of antibodies, immunoglobulin-binding proteins and polypeptides derived from non-human species (such as mice or rats) to the human body, while still retaining the antigen-binding characteristics of the original antibodies. For example, it is feasible to use CDR transplants (Jones et al., Nature 321:522 (1986)) and variants thereof; including "reshaping", (Verhoeyen, et al., 1988 Science 239:1534-1536; Riechmann, et al., 1988 Nature 332:323-337; Tempest, et al., Bio/Technol 1991 9:266-271), "hyperchimerization", (Queen, et al., 1989 Proc Natl Acad Sci USA 86:10029-10033; Co, et al., 1991 Proc Natl Acad Sci USA 88:2869-2873; Co, et al., 1992 J Immunol 148:1149-1154), and "veneering", (Mark, et al., "Derivation of therapeutically active humanized and veneered anti-CD18 antibodies." In: Metcalf B W, Dalton B J, eds. Cellular adhesion: molecular definition to therapeutic potential. New York: Plenum Press, 1994: 291-312) and other technical means to humanize the binding domain of non-human sources. If other regions, such as the hinge region and the constant region domain, are also derived from non-human sources, these regions can also be humanized.

In the present application, the term "fully human antibody" generally refers to antibodies in which all the parts (including the constant region, CH and/or CL regions of the antibody) are encoded by genes of human origin. Fully human antibodies can significantly reduce the immune side effects caused by heterologous antibodies in the human body.

In the present application, the term "bispecific antibody (BsAb)" generally refers to antibodies or antibody constructs that have dual specificity in their binding arms. Naturally occurring antibodies are monospecific and have the same specificity in their antigen-binding arms. Bispecific antibodies are obtained from two different sources and constructed through recombinant DNA or cell fusion techniques, enabling them to retain the two specificities of the original antibodies. Bispecific antibodies carry two different antigen-binding sites. The Fc part of a bispecific antibody is different from that of any parental monospecific antibody. Since bispecific antibodies have dual specificity for both drugs and tumor cells, theoretically, they can lead to a higher accumulation of drugs in tumors than in tumor-free areas of the body. Most bispecific new antibodies can redirect cytotoxic effector cells (such as Tc cells, NK cells, neutrophils) to targeted cells (such as tumor cells). Bispecific antibodies can simultaneously block two different mediators/pathways that play unique or overlapping functions in the pathogenesis, and they may also increase the binding specificity by interacting with two different cell surface antigens rather than one. (Fanger, Michael W., and Paul M. Guyre. "Bispecific antibodies for targeted cellular cytotoxicity." Trends in biotechnology, 1991: 375-380), (Fan, Gaowei, et al. "Bispecific antibodies and their applications." Journal of hematology & oncology, 2015: 130).

In the present application, the term "IgG" refers to polypeptides belonging to a class of antibodies that are essentially encoded by the recognized immunoglobulin $\gamma$ gene. In humans, this class includes IgG1, IgG2, IgG3, and IgG4. In mice, this class includes IgG1, IgG2a, IgG2b, and IgG3.

In the present application, the term "chimeric antigen receptor" (CAR) generally refers to a recombinant polypeptide including at least an extracellular domain, a transmembrane region, and an intracellular domain that specifically binds to an antigen or a target. For example, a hinge region is included between the extracellular domain and the transmembrane region. For example, the chimeric antigen receptor may further include a low-density lipoprotein receptor-related protein or a fragment thereof. For example, the chimeric antigen receptor may include a signal peptide. The binding of the extracellular domain of CAR to the target antigen on the surface of target cells leads to CAR clustering and delivery of activation stimulus to the CAR-containing cells. CAR redirects the specificity of immune effector cells and triggers proliferation, cytokine production, phagocytosis and/or production of molecules capable of mediating the death of cells expressing target antigens in a manner that is not dependent on major histocompatibility (MHC).

In the present application, the terms "polypeptide", "peptide", and "protein" are used interchangeably herein and refer to a polymer of amino acid residues. This term may be used to refer to amino acid polymers in which one or more amino acid residues are synthetic chemical mimics of their corresponding naturally occurring amino acids, as well as to naturally occurring amino acid polymers, those amino acid polymers containing modified residues, and non-naturally occurring amino acid polymers. For example, the polypeptide may include the antigen-binding protein.

In the present application, the term "nucleic acid molecule" generally refers to an isolated nucleotide, deoxyribonucleotide or ribonucleotide of any length or an analogue thereof that has been isolated from its natural environment or synthesized artificially.

In the present application, the term "vector" generally refers to a nucleic acid molecule capable of self-replication in a suitable host, which transfers the inserted nucleic acid molecule into and/or between host cells. The vector may include a vector mainly used for inserting DNA or RNA into cells, a vector mainly used for replicating DNA or RNA, and a vector mainly used for expression of DNA or RNA transcription and/or translation. The vector also includes a vector having multiple of the above functions. The vector may be a polynucleotide that can be transcribed and translated into a polypeptide when introduced into a suitable host cell. Generally, the vector can produce the desired expression products by culturing suitable host cells containing the vector.

In the present application, the term "cell" may include both modified cells and unmodified cells. For example, unmodified cells may be naturally derived cells, while modified cells may be those for which nucleic acids have been transfected. The term "cell" includes prokaryotic cells used for plasmid propagation and eukaryotic cells used for nucleic acid expression and the production of encoded polypeptides. For example, a cell may include the antigen-binding protein, the nucleic acid molecule and/or the vector. For another example, the cells may be modified or unmodified immune effector cells. The term "immune effector cell" generally refers to immune cells that participate in immune responses and perform effector functions. For example, the exercise of effector functions may include the clearance of foreign antigens or the promotion of immune effector responses. For example, the immune effector cells may include T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells. For example, the immune effector cell may be a T cell.

In the present application, the term "specifically bind to" or "specific" generally refers to measurable and reproducible interactions, such as the binding between targets and antibodies, which can determine the presence of the targets in the presence of a heterogeneous population of molecules, including biomolecules. For example, an antibody that specifically binds to a target (which may be an epitope) may be an antibody that binds to the target with greater affinity, avidity, more easily, and/or for a greater duration than it binds to other targets. In some embodiments, the antibody specifically binds to the epitope on the protein, and the epitope is conserved among proteins of different species. In some embodiments, specific binding may include but does not require exclusive binding.

In the present application, the term "subject" generally refers to human or non-human animals, including but not limited to cats, dogs, horses, pigs, cows, sheep, rabbits, mice, rats, or monkeys.

In the present application, the protein, polypeptide and/or amino acid sequences involved should also be understood to include at least the following ranges: variants or homologs having the same or similar functions as those of the protein or polypeptide.

In the present application, the variants may be, e.g., proteins or polypeptides with one or more amino acid substitutions, deletions or additions in the amino acid sequence of the protein and/or the polypeptide (e.g., specifically binding to CD39). For example, the functional variants may include proteins or polypeptides with amino acid changes by at least 1, for example, 1-30, 1-20 or 1-10, and further for example, 1, 2, 3, 4 or 5 amino acid substitutions, deletions and/or insertions. The functional variants can substantially maintain the biological properties of the protein or the polypeptide before changes (e.g., substitution, deletion or addition). For example, the functional variants can maintain at least 60%, 70%, 80%, 90%, or 100% of the biological activity (e.g., antigen-binding ability) of the protein or the polypeptide before change. For example, the substitution may be a conservative substitution.

In the present application, the homolog may be a protein or polypeptide having at least about 85% (e.g., at least about 85%, about 90%, about 91%, about 92%, about 93%, about 94%, about 95%, about 96%, about 97%, about 98%, about 99% or higher) sequence homology with the amino acid sequence of the protein and/or the polypeptide (for example, specifically binding to CD39).

In the present application, the homology generally refers to the similarity, likeness or correlation between two or more sequences. The "percentage of sequence homology" can be calculated by: comparing two sequences to be aligned in a comparison window to determine the number of positions where the same nucleic acid bases (e.g., A, T, C, G, I) or the same amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are present in both sequences so as to obtain the number of matching positions, dividing the number of matching positions by the total number of positions in the comparison window (i.e., window size), and multiplying the result by 100, to generate the percentage of sequence homology. The alignment for determining the percentage of sequence homology can be achieved in a variety of ways known in the art, for example, by using publicly available computer software, such as BLAST, BLAST-2, ALIGN or Megalign (DNASTAR) software. A person skilled in the art can determine appropriate parameters for aligning sequences, including any algorithms needed to achieve the maximum alignment over the full-length sequence range being compared or within the target sequence region. The homology can also be determined by the following methods: FASTA and BLAST. A description of FASTA algorithm can be found in "Improved tools for biological sequence comparison" by W. R. Pearson and D. J. Lipman, Proc. Natl. Acad. Sci., 85: 2444-2448, 1988; and "Rapid and Sensitive Protein Similarity Searches" by D. J. Lipman and W. R. Pearson, Science, 227: 1435-1441, 1989. A description of BLAST algorithm can be found in "Basic Local Alignment Search Tool" by S. Altschul, W. Gish, W. Miller, E. W. Myers and D. Lipman, Journal of Molecular Biology, 215: 403-410, 1990.

In the present application, the term "include" generally refers to the meaning of include, encompass, contain or embrace. In some cases, it also indicates the meaning of "is" or "composed of".

In the present application, the term "about" generally refers to varying in a range of 0.5%-10% above or below a specified value, for example, varying in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below a specified value.

**Detailed Description of the Invention**

**Antibody or antigen-binding fragment thereof**

In one aspect, the present application provides an antibody or an antigen-binding fragment thereof, which binds to a CD39 protein at a $K_D$ value of $1\times10^{-8}$ M or below (e.g., the $K_D$ value is not higher than about $5\times10^{-9}$ M, not higher than about $3\times10^{-9}$ M, not higher than about $1\times10^{-9}$ M, not higher than about $9\times10^{-10}$ M, not higher than about $8\times10^{-10}$ M, not higher than about $7\times10^{-10}$ M, not higher than about $6\times10^{-10}$ M, not higher than about $5\times10^{-10}$ M, not higher than about $4\times10^{-10}$ M, not higher than about $3\times10^{-10}$ M, not higher than about $2\times10^{-10}$ M, not higher than $1\times10^{-10}$ M, not higher than about $9\times10^{-11}$ M, not higher than about $8\times10^{-11}$ M, not higher than about $7\times10^{-11}$ M, not higher than about $6\times10^{-11}$ M, not higher than about $1\times10^{-11}$ M or below).

The antibody or the antigen-binding fragment thereof as described in the present application can specifically bind to a CD39 protein.

In some embodiments, the antibody targeting CD39 or the antigen-binding fragment thereof as described in the present application can block the ATPase activity of CD39, reduce the production of adenosine, maintain the ATP level in the tumor microenvironment, and promote the activation of T cells by dendritic cells.

In some embodiments, the antibody targeting CD39 or the antigen-binding fragment thereof as described in the present application can reduce the immunosuppressive function of regulatory T cells and reactivate exhaustion T cells.

In the present application, the antigen-binding protein may include an antibody or an antigen-binding fragment thereof. In the present application, the antigen-binding fragment may include Fab, Fab', F(ab)$_2$, an Fv fragment, F(ab')$_2$, scFv, di-scFv, VHH and/or dAb. In the present application, the antibody may include a monoclonal antibody, a chimeric antibody, a humanized antibody, and a fully human antibody.

In the present application, the antibody may be a single-domain antibody. In the present application, the antigen-binding fragment may be a VHH.

In the present application, when defining the amino acid sequence of the antibody or the antigen-binding fragment thereof, the variant of the antibody or the antigen-binding fragment thereof is included. In the present application, the variant may be an amino acid sequence that has substantially the same function (e.g., capable of specifically binding to CD39) as the antibody or the antigen-binding fragment thereof and has a sequence identity therewith of at least 85% or above (e.g., at least about 85%, about 90%, about 91%, about 92%, about 93%, about 94%, about 95%, about 96%, about 97%, about 98%, about 99% or higher). In some embodiments, the variant of the amino acid sequence is an amino acid sequence that has substantially the same function as the amino acid sequence (e.g., capable of specifically binding to PD-L1) and includes one or more (e.g., 1-2, 1-3, 1-4, 1-5, 1-6, 1-7, 1-8, 1-9, 1-10 or more) amino acid additions, deletions or replacements on this basis. For another example, the variant in the present application may be selected from a group consisting of: proteins or polypeptides obtained through substituting, deleting or adding one or more amino acids in the antibody or the antigen-binding fragment thereof; and proteins or polypeptides having a sequence homology of 85% or above (e.g., at least about 85%, about 90%, about 91%, about 92%, about 93%, about 94%, about 95%, about 96%, about 97%, about 98%, about 99% or higher) with the antibody or the antigen-binding fragment thereof.

In the present application, the antibody or the antigen-binding fragment thereof may include at least one CDR in an antibody heavy chain variable region (VH), and the VH includes an amino acid sequence as set forth in any one of SEQ ID NO: 8, SEQ ID NO: 16, SEQ ID NO: 21, SEQ ID NO: 27, SEQ ID NO: 32, SEQ ID NO: 37, and SEQ ID NO: 41.

A CDR of an antibody, also known as a complementary determining region, is a part of a variable region. Amino acid residues in this region can be in contact with antigens or antigen epitopes. The CDR of an antibody can be determined through a variety of numbering systems, such as CCG, Kabat, Chothia, IMGT, AbM, or a combination of Kabat/Chothia, etc. These numbering systems are known in the art, particularly see, e.g., http://www.bioinf.org.uk/abs/index.html#ka batnum. Those skilled in the art can determine the CDR regions using different numbering systems based on the sequence and structure of the antibody. There may be differences among the CDR regions when using different numbering systems. In the present application, the CDR encompasses CDR sequences divided according to any CDR division method; it also encompasses variants thereof, wherein the variants include substitution, deletion and/or addition of one or more amino acids in the amino acid sequence of the CDR, for example, substitution, deletion and/or insertion of 1-30, 1-20 or 1-10, further for example, 1, 2, 3, 4, 5, 6, 7, 8 or 9 amino acids; and it also encompasses homologs thereof, wherein the homologs may be amino acid sequences that have a sequence homology of at least about 85% (e.g., at least about 85%, about 90%, about 91%, about 92%, about 93%, about 94%, about 95%, about 96%, about 97%, about 98%, about 99% or higher) with the amino acid sequence of the CDR. In the present application, the isolated antigen-binding protein is defined by the IMGT numbering system.

In the present application, the antibody or the antigen-binding fragment thereof may include an HCDR3, wherein the HCDR3 may include an amino acid sequence as set forth in any one of SEQ ID NO: 3, SEQ ID NO: 54, and SEQ ID NO: 55. For example, the HCDR3 may include an amino acid sequence as set forth in any one of SEQ ID NO: 3, SEQ ID NO: 11, SEQ ID NO: 18, SEQ ID NO: 24, SEQ ID NO: 29, and SEQ ID NO: 33.

In the present application, the antibody or the antigen-binding fragment thereof may include an HCDR2, wherein the HCDR2 may include an amino acid sequence as set forth in any one of SEQ ID NO: 2, SEQ ID NO: 10, SEQ ID NO: 23, and SEQ ID NO: 28.

In the present application, the antibody or the antigen-binding fragment thereof may include an HCDR1, wherein the HCDR1 may include an amino acid sequence as set forth in any one of SEQ ID NO: 1, SEQ ID NO: 53, and SEQ ID NO: 22. For example, the HCDR1 may include an amino acid sequence as set forth in any one of SEQ ID NO: 1, SEQ ID NO: 9, SEQ ID NO: 17, and SEQ ID NO: 22.

In the present application, the antibody or the antigen-binding fragment thereof may include an HCDR1, an HCDR2, and an HCDR3.

For example, the HCDR1 includes an amino acid sequence as set forth in SEQ ID NO: 1, the HCDR2 includes an amino acid sequence as set forth in SEQ ID NO: 2, and the HCDR3 includes an amino acid sequence as set forth in SEQ ID NO: 3.

For example, the HCDR1 includes an amino acid sequence as set forth in SEQ ID NO: 9, the HCDR2 includes an amino acid sequence as set forth in SEQ ID NO: 10, and the HCDR3 includes an amino acid sequence as set forth in SEQ ID NO: 11.

For example, the HCDR1 includes an amino acid sequence as set forth in SEQ ID NO: 17, the HCDR2 includes an amino acid sequence as set forth in SEQ ID NO: 10, and the HCDR3 includes an amino acid sequence as set forth in SEQ ID NO: 18.

For example, the HCDR1 includes an amino acid sequence as set forth in SEQ ID NO: 22, the HCDR2 includes an amino acid sequence as set forth in SEQ ID NO: 23, and the HCDR3 includes an amino acid sequence as set forth in SEQ ID NO: 24.

For example, the HCDR1 includes an amino acid sequence as set forth in SEQ ID NO: 22, the HCDR2 includes an amino acid sequence as set forth in SEQ ID NO: 28, and the HCDR3 includes an amino acid sequence as set forth in SEQ ID NO: 29.

For example, the HCDR1 includes an amino acid sequence as set forth in SEQ ID NO: 22, the HCDR2 includes an amino acid sequence as set forth in SEQ ID NO: 28, and the HCDR3 includes an amino acid sequence as set forth in SEQ ID NO: 33.

In the present application, the antibody or the antigen-binding fragment thereof may include an H-FR1, an H-FR2, an H-FR3, and an H-FR4.

For example, the H-FR1 may include an amino acid sequence as set forth in SEQ ID NO: 56, the H-FR2 may include an amino acid sequence as set forth in SEQ ID NO: 57, the H-FR3 may include an amino acid sequence as set forth in SEQ ID NO: 58, and the H-FR4 may include an amino acid sequence as set forth in SEQ ID NO: 59.

For example, the H-FR1 may include an amino acid sequence as set forth in any one of SEQ ID NO: 4, SEQ ID NO: 12, SEQ ID NO: 19, SEQ ID NO: 34, and SEQ ID NO: 38, the H-FR2 may include an amino acid sequence as set forth in any one of SEQ ID NO: 5, SEQ ID NO: 13, SEQ ID NO: 20, SEQ ID NO: 25, SEQ ID NO: 30, and SEQ ID NO: 35, the H-FR3 may include an amino acid sequence as set forth in any one of SEQ ID NO: 6, SEQ ID NO: 14, SEQ ID NO: 26, SEQ ID NO: 31, SEQ ID NO: 36, and SEQ ID NO: 40, and the H-FR4 includes an amino acid sequence as set forth in any one of SEQ ID NO: 7, SEQ ID NO: 15, and SEQ ID NO: 39.

In the present application, the antibody or the antigen-binding fragment may be a VHH. For example, the VHH may include an amino acid sequence as set forth in any one of SEQ ID NO: 8, SEQ ID NO: 16, SEQ ID NO: 21, SEQ ID NO: 27, SEQ ID NO: 32, SEQ ID NO: 37, and SEQ ID NO: 41.

In the present application, the antibody or the antigen-binding fragment thereof may include a heavy chain constant region. For example, the heavy chain constant region refers to a region containing at least three heavy chain constant domains, CH1, CH2, and CH3. Non-limiting exemplary heavy chain constant regions include $\gamma$, $\delta$, and $\alpha$. Non-limiting exemplary heavy chain constant regions also include $\varepsilon$ and $\mu$. Each heavy chain constant region corresponds to one antibody isotype. For example, an antibody containing a $\gamma$ constant region is an IgG antibody, an antibody containing a $\delta$ constant region is an IgD antibody, and an antibody containing an $\alpha$ constant region is an IgA antibody. In addition, an antibody containing a $\mu$ constant region is an IgM antibody, and an antibody containing an $\varepsilon$ constant region is an IgE antibody. Some isotypes can be further subdivided into subclasses. For example, IgG antibodies include, but are not limited to, IgG1 (containing a $\gamma_1$ constant region), IgG2 (containing a $\gamma_2$ constant region), IgG3 (containing a $\gamma_3$ constant region), and IgG4 (containing a $\gamma_4$ constant region) antibodies; IgA antibodies include, but are not limited to, IgA1 (containing an $\alpha_1$ constant region) and IgA2 (containing an $\alpha_2$ constant region) antibodies; and IgM includes, but is not limited to, IgM1 and IgM2.

In the present application, the antibody heavy chain constant region may be derived from IgG. In the present application, the isolated antigen-binding protein may include an antibody heavy chain constant region, and the antibody heavy chain constant region may be derived from a human IgG. In the present application, the isolated antigen-binding protein may include an antibody heavy chain constant region, and the antibody heavy chain constant region may be derived from IgG4. In the present application, the heavy chain constant region of the antigen-binding protein may include an Fc region of IgG. For example, the antibody heavy chain constant region may include an amino acid sequence as set forth in SEQ ID NO: 44.

### Bispecific antibody or antigen-binding fragment thereof

In another aspect, the present application also provides a bispecific antibody or an antigen-binding fragment thereof, which includes a first binding domain that specifically binds to a CD39 protein, wherein the first binding domain includes the antibody targeting CD39 or the antigen-binding fragment thereof in the present application. That is, the antibody targeting CD39 or an antigen-binding fragment thereof as described in the present application can also form a bispecific antibody structure with antibodies targeting other targets.

In the present application, the bispecific antibody or the antigen-binding fragment thereof includes the VHH that specifically binds to CD39 as described in the present application.

In the present application, the second binding domain of the bispecific antibody or the antigen-binding fragment thereof can target PD-L1. For example, the second binding domain may be an antibody targeting PD-L1 or an antigen-binding fragment thereof.

In the present application, the PD-L1 antibody or the antigen-binding fragment thereof may include an HCDR1, an HCDR2, and an HCDR3, wherein the HCDR1 may include an amino acid sequence as set forth in SEQ ID NO: 45, the HCDR2 may include an amino acid sequence as set forth in SEQ ID NO: 46, and the HCDR3 may include an amino acid sequence as set forth in SEQ ID NO: 47. In the present application, the PD-L1 antibody or the antigen-binding fragment thereof may include an LCDR1, an LCDR2, and an LCDR3, wherein the LCDR1 may include an amino acid sequence as set forth in SEQ ID NO: 49, the LCDR2 may include an amino acid sequence as set forth in SEQ ID NO: 50(GNS), and the LCDR3 may include an amino acid sequence as set forth in SEQ ID NO: 51.

In the present application, the PD-L1 antibody or the antigen-binding fragment thereof may include a VH, wherein the VH may include an amino acid sequence as set forth in SEQ ID NO: 48. In the present application, the PD-L1 antibody or the antigen-binding fragment thereof may include a VL, wherein the VL includes an amino acid sequence as set forth in SEQ ID NO: 52.

In the present application, the PD-L1 antibody or the antigen-binding fragment thereof may include an antibody heavy chain constant region and an antibody light chain constant region.

In the present application, the CD39-targeting moiety of the bispecific antibody or the antigen-binding fragment thereof can be linked to the PD-L1-targeting moiety directly or indirectly. For example, the N-terminus of the CD39-targeting moiety can be linked to the C-terminus of the PD-L1 targeting moiety directly or indirectly. For example, the C-terminus of the CD39-targeting moiety can be linked to the N-terminus of the PD-L1 targeting moiety directly or indirectly. For example, the linkage may be through a linker. For example, the linker may include an amino acid sequence as set forth in (G4S)n, wherein n is a positive integer from 1 to 10. For example, n is 3.

In the present application, the bispecific antibody or the antigen-binding fragment thereof may include a first polypeptide chain and a second polypeptide chain, wherein the first polypeptide chain may include a heavy chain variable region of the PD-L1 antibody or the antigen-binding fragment thereof, a heavy chain constant region of the PD-L1 antibody or the antigen-binding fragment thereof and the VHH that specifically binds to CD39, and the second polypeptide chain may include a light chain variable region and a light chain constant region of the PD-L1 antibody or the antigen-binding fragment thereof.

In the present application, the bispecific antibody or the antigen-binding fragment thereof may include a first polypeptide chain and a second polypeptide chain, wherein the first polypeptide chain may include the heavy chain variable region of the PD-L1 antibody or the antigen-binding fragment thereof, the heavy chain constant region of the PD-L1 antibody or the antigen-binding fragment thereof, a linker and the VHH that specifically binds to CD39, and the second polypeptide chain may include the light chain variable region and the light chain constant region of the PD-L1 antibody or the antigen-binding fragment thereof.

In the present application, the bispecific antibody or the antigen-binding fragment thereof may include a first polypeptide chain and a second polypeptide chain, wherein the first polypeptide chain may include, sequentially from N-terminus to C-terminus: the heavy chain variable region of the PD-L1 antibody or the antigen-binding fragment thereof, the heavy chain constant region of the PD-L1 antibody or the antigen-binding fragment thereof, a linker and the VHH that specifically binds to CD39, and the second polypeptide chain includes the light chain variable region and the light chain constant region of the PD-L1 antibody or the antigen-binding fragment thereof.

In the present application, the bispecific antibody or the antigen-binding fragment thereof may include a first polypeptide chain and a second polypeptide chain, wherein the first polypeptide chain may include, sequentially from N-terminus to C-terminus: the VHH that specifically binds to CD39, a linker, the heavy chain variable region of the PD-L1 antibody or the antigen-binding fragment thereof and the heavy chain constant region of the PD-L1 antibody or the antigen-binding fragment thereof, and the second polypeptide chain may include the light chain variable region and the light chain constant region of the PD-L1 antibody or the antigen-binding fragment thereof.

In the present application, the bispecific antibody or the antigen-binding fragment thereof may include two first polypeptide chains and two second polypeptide chains.

**CAR-T cells autocrinely secrete CD39 antigen-binding protein**

In another aspect, the CAR-T cells can also autocrinely secrete the CD39-targeting antigen-binding protein as described in the present application to enhance the proliferation effects and/or tumor cell-killing effects of the CAR-T cells. For example, by designing cleavable peptides, the sequence encoding the CAR and the sequence encoding the CD39 antibody may be designed into the same vector, thereby enabling the CAR-T cells to autocrinely secrete the CD39 antibody of the present application. In the present application, the CAR may be a CAR targeting MSLN. For example, the CAR may include a signal peptide, an antigen-binding protein targeting MSLN, a hinge region, a transmembrane region, a costimulatory region and/or an intracellular signaling region. For example, the signal peptide may be a CD8 signal peptide, for example, the CD8 signal peptide may include an amino acid sequence as set forth in SEQ ID NO: 66. For example, the antigen-binding protein targeting MSLN may be a VHH, for example, the VHH may include an amino acid sequence as set forth in SEQ ID NO: 67. For example, the hinge region may be a CD8 hinge region, for example, the CD8 hinge region may include an amino acid sequence as set forth in SEQ ID NO: 68. For example, the transmembrane region may be a CD8 transmembrane region, for example, the CD8 transmembrane region may include an amino acid sequence as set forth in SEQ ID NO: 69. For example, the costimulatory region may be a 4-1BB costimulatory region, for example, the 4-1BB costimulatory region may include an amino acid sequence as set forth in SEQ ID NO: 70. For example, the intracellular signaling region may be a CD3ζ intracellular signaling region, for example, the CD3ζ intracellular signaling region may include an amino acid sequence as set forth in SEQ ID NO: 71.

**Polypeptide, nucleic acid molecule, vector, cell, drug combination, pharmaceutical composition, preparation method**

In another aspect, the present application also provides a polypeptide, which includes the antibody or the antigen-binding fragment thereof, or the bispecific antibody or the antigen-binding fragment thereof.

In another aspect, the present application also provides one or more isolated nucleic acid molecules, which can encode the antibody or the antigen-binding fragment thereof, or the bispecific antibody or the antigen-binding fragment thereof as described in the present application. For example, each of the one or more nucleic acid molecules can encode the whole antibody or the antigen-binding fragment thereof, or a part thereof (e.g., one or more of HCDR1-3, LCDR1-3, VL, VH, light chains or heavy chains).

The nucleic acid molecule of the present application may be isolated. For example, they can be produced or synthesized by the following methods: (i) amplified *in vitro,* e.g., produced by amplification through polymerase chain reaction (PCR), (ii) produced by cloning and recombination, (iii) purified, for example, by enzymatic digestion and gel electrophoresis fractionation, or (iv) synthesized, for example through chemical synthesis. In some embodiments, the isolated nucleic acid is a nucleic acid molecule prepared by a recombinant DNA technology.

In the present application, the nucleic acids encoding the antibody or the antigen-binding fragment thereof can be prepared by a variety of methods known in the art, including but not limited to, restriction fragment manipulation or overlap-extension PCR using synthetic oligonucleotides. Detailed procedures can be found in Sambrook et al., Molecular Cloning, A Laboratory Manual, Cold Spring Harbor Laboratory Press, Cold Spring Harbor, N.Y., 1989; and Ausube et al., Current Protocols in Molecular Biology, Greene Publishing and Wiley-Interscience, New York N.Y., 1993.

In the present application, the nucleic acid molecule may also include a nucleic acid molecule encoding other proteins. For example, the other proteins may be CARs.

In another aspect, the present application provides one or more vectors, which include the one or more nucleic acid molecules of the present application. Each vector may include one or more of the nucleic acid molecules. In addition, the vector may further include other genes, e.g., a marker gene that allows the selection of the vector in an appropriate host cell and under appropriate conditions. In addition, the vector may further include an expression control element that allows the coding region to be properly expressed in an appropriate host. Such control elements are well known by those skilled in the art. For example, they may include a promoter, a ribosome binding site, an enhancer and other control elements that regulate gene transcription or mRNA translation, and the like. In some embodiments, the expression control sequence is an adjustable element. The specific structure of the expression control sequence may vary depending on the function of the species or cell type, but typically includes 5' non-transcriptional sequences and 5' and 3' non-translational sequences involved in the initiation of transcription and translation, respectively, such as TATA box, capped sequences, CAAT sequences and the like. For example, 5' non-transcriptional expression control sequences may include a promoter region which may include a promoter sequence for transcriptional control of a functionally linked nucleic acid. The one or more nucleic acid molecules of the present application may be operatively linked to the expression control element.

The vector may include, e.g., plasmid, cosmid, virus, phage, or other vectors commonly used in, e.g., genetic engineering. For example, the vector is an expression vector.

In the present application, the vector may also include the nucleic acid molecule encoding other proteins. For example, the vector may also include a nucleic acid molecule encoding a chimeric antigen receptor (CAR). For example, the CAR may target any known targets. For example, the CAR may target MSLN (mesothelin).

In another aspect, the present application provides a cell, which may include the antibody or the antigen-binding fragment thereof of the present application, the bispecific antibody or the antigen-binding fragment thereof of the present application, the nucleic acid molecule of the present application and/or the vector of the present application. The cells may be host cells. In some embodiments, each kind of or each of the host cells may include one or one kind of the nucleic acid molecule or the vector of the present application. In some embodiments, each kind of or each of the host cells may include multiple (e.g., two or more) or multiple kinds of (e.g., two or more kinds of) the nucleic acid molecule or the vector of the present application. For example, the vector of the present application can be introduced into the host cells, e.g., eukaryotic cells, such as plant-derived cells, fungi or yeast cells, and the like. The vector of the present application can be introduced into the host cells by methods known in the art, such as electroporation, Lipofectine transfection, Lipofectamin transfection, and the like.

In the present application, the cells may include immune cells. In some embodiments, the cells may include T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells.

In the present application, the cells may express the antibody or the antigen-binding fragment thereof of the present application, or the bispecific antibody or the antigen-binding fragment thereof of the present application. In some embodiments, the cells may also include and/or express other proteins. For example, the cells may also include and/or express CARs.

In another aspect, the present application also provides a drug combination, which may include the following components: (1) the antibody or the antigen-binding fragment thereof of the present application, or the bispecific antibody or the antigen-binding fragment thereof of the present application; and (2) a cell. For example, the cell may be a modified cell or an unmodified cell. For example, the cell may be an immune cell. For example, the cell may be a T cell. In the drug combination, components (1) and (2) can be placed separately or mixed to form a mixture. Component (1) can be conducive to improving the function of component (2), for example, promoting the proliferation of component (2).

In another aspect, the present application also provides a pharmaceutical composition, which may include the antibody or the antigen-binding fragment thereof, the bispecific antibody or the antigen-binding fragment thereof, the nucleic acid molecule, the vector, the cell and/or the drug combination, and optionally a pharmaceutically acceptable carrier.

In some embodiments, the pharmaceutical composition may include one or more (pharmaceutically effective) carriers, stabilizers, excipients, diluents, solubilizers, surfactants, emulsifiers and/or preservatives, and other suitable preparations. The acceptable ingredients of the composition are preferably non-toxic to the recipient at the dosage and concentration used. The pharmaceutical composition of the present application may include liquid, frozen and lyophilized compositions.

In some embodiments, the pharmaceutically acceptable carrier may include any and all solvents, dispersion media, coatings, isotonic agents and absorption delaying agents that are compatible with the medication, which are generally safe, non-toxic and neither biologically nor otherwise undesirable.

In some embodiments, the pharmaceutical composition may be administered parenterally, transdermally, intracavity, intraarterially, intrathecally and/or intranasally or directly injected into tissues. For example, the pharmaceutical composition may be administered to patients or subjects by means of infusion or injection. In some embodiments, the administration of the pharmaceutical composition can be done in different ways, such as intravenously, intraperitoneally, subcutaneously, intramuscularly, locally, or intradermally.

In another aspect, the present application also provides a method for preparing the antibody or the antigen-binding fragment thereof and/or the bispecific antibody or the antigen-binding fragment thereof. The method may include culturing the cell under conditions enabling the expression of the antibody or the antigen-binding fragment thereof and/or the bispecific antibody or the antigen-binding fragment thereof.

Use

In another aspect, the present application also provides a use of the antibody or the antigen-binding fragment thereof, the bispecific antibody or the antigen-binding fragment thereof, the nucleic acid molecule, the cell, the drug combination, or the pharmaceutical composition in the preparation of a drug for preventing and/or treating a disease and/or a disorder.

In another aspect, the present application also provides a method for preventing and/or treating a disease and/or a disorder, which includes administering to a subject in need thereof the antibody or the antigen-binding fragment thereof, the bispecific antibody or the antigen-binding fragment thereof, the nucleic acid molecule, the cell, the drug combination, or the pharmaceutical composition.

In another aspect, the present application also provides the antibody or the antigen-binding fragment thereof, the bispecific antibody or the antigen-binding fragment thereof, the nucleic acid molecule, the cell, the drug combination, or the pharmaceutical composition, which are used for preventing and/or treating a disease and/or a disorder.

In some embodiments, the disease and/or disorder may include a tumor.

In some embodiments, the tumor may include a solid tumor and/or a hematological tumor.

In some embodiments, the tumor may include a tumor affected by CD39 activity. For example, the tumor may be a CD39-overexpressing tumor. For example, the tumor may be colon cancer.

In the present application, the subject may include human or non-human animals.

In another aspect, the present application provides a kit or a drug delivery device, which includes the antibody or the antigen-binding fragment thereof, the bispecific antibody or the antigen-binding fragment thereof, the nucleic acid molecule, the cell, the drug combination, or the pharmaceutical composition. The kit can be used for detecting the presence and/or content of CD39.

Without intending to be limited by any theory, the following examples are only to illustrate the antibody or the antigen-binding fragment thereof, the preparation method, and the use of the present application, and are not intended to limit the scope of the present application.

**EXAMPLES**

**Example 1 Screening of Nanobodies Targeting human CD39**

1.1 Alpaca Immunization and Construction of Phage-Display Immune Nanobody Library

Human CD39-His protein (CD9-H52H4) was purchased from ACRO Biosystems and, following well-known procedures in the art, NBbiolab was commissioned to perform immunization of alpacas and library construction. The initial immunization dose was 0.5 mg of protein, and 0.25 mg of protein was used for each of the subsequent three immunizations. After the third and fourth immunizations, 50 mL of peripheral blood was collected respectively, and a small amount of serum was separated for ELISA to evaluate the immune effects. The titers after the third and fourth immunizations both reached above 1:16,000 (coating antigen at 2 $\mu$g/mL; OD positive value > 2.0), meeting the requirements for library construction. PBMCs were isolated, and total RNA was extracted from the PBMCs of the third and fourth immunizations using the RNAiso Plus reagent. The extracted RNA was reverse-transcribed into cDNA using the PrimeScript™ II 1st Strand cDNA Synthesis Kit (6210A, Takara). Following the protocols known in the art, the variable domain of the heavy chain antibody (VHH) was amplified by two rounds of nested PCR, and the target fragments were recovered from gels and cloned into the phage-display vector pComb3XSS using the restriction enzyme SfiI. After desalting, the plasmids were electroporated into electrocompetent *E. coli* TG1 cells to construct the phage-display nanobody library, which was subsequently evaluated for diversity. The antibody library was diluted in gradients and plated to calculate the library size to be $1.30 \times 10^9$. Forty-eight monoclones were then randomly selected for sequencing. The results showed that all were correctly inserted. Except for five monoclones identified as non-monoclones, the CDRs of the remaining sequences were not completely consistent. The sense mutation rate was determined to be 89.6% (43/48), and the diversity was $1.16 \times 10^9$.

1.2 Panning of Antibody Sequences Targeting Human CD39

10 $\mu$g/mL of human CD39-His protein (CD9-H52H4, ACRO) was coated onto plates and placed at 4°C overnight. The next day, the plates were washed three times with 1× PBST (PBS containing 0.05% Tween-20), and then blocked with 0.5% BSA at room temperature for 2 hours. After three additional washes with 1× PBST, 100 $\mu$L of phages (derived from the phage-display immune nanobody library constructed in Example 1) were added for screening. After 1 hour, the plates were washed 15 times with 1× PBST to remove phages that did not bind to antigens. Finally, the bound phages were eluted with Glycine-HCl at pH 2.2 (100 $\mu$L/well) and neutralized with Tris-HCl at pH 8.0. Half of the eluted phages were used to infect TG1 in the logarithmic growth phase, which was superinfected with M13KO7 after half an hour and cultured overnight. The phages were precipitated the next day and used for the subsequent round of screening. A similar screening procedure was repeated for three rounds. In the second round, 5 $\mu$g/mL of human CD39-His was used for positive screening, followed by 30 washes with 1× PBST. In the third round, 1 $\mu$g/mL of human CD39-His was used for positive screening, followed by 50 washes with 1× PBST. After the third round, pool ELISA was performed to detect the specific enrichment of antibodies.

1.3 Screening of Specific Monoclonal Antibodies Using Phage-Based Enzyme-Linked Immunosorbent Assay (ELISA)

Single colonies were picked into twelve 96-well deep-well plates respectively and cultured overnight to produce phages. The next day, human CD39-His, IgG1-Fc and 0.2% BSA were coated onto 384-well plates. After washing three times with 1×PBST (PBS containing 0.05% Tween 20), the plates were blocked with 0.5% BSA at room temperature for 2 hours. After three additional washes with 1× PBST, 100 $\mu$L of phage supernatant was added and incubated for 1 hour. After washing, anti-M13-HRP (SinoBiological, Cat. No. 11973-MM05T-H) was added and incubated for 1 hour. After washing, TMB chromogen solution (Invitrogen, Cat. No. 002023) was added. After 3-5 minutes, 2 M phosphoric acid was added to terminate the chromogenic reaction. Finally, OD450 was read. When the OD value of a sample well is more than 5 times that of the control well, it is regarded as a positive well. The amino acid sequences of various clones were analyzed using sequence alignment software, obtaining a total of 419 positive sequences. According to the differences in CDR3, 90 candidate sequences with larger CDR differences were selected for subsequent expression identification.

**Example 2 Evaluation of *In Vitro* Affinity and Enzymatic Activity Inhibition Function of CD39 Nanobodies**

2.1 Expression and Purification of Bivalent Nanobodies in Expi293F

The nucleotide sequences of 90 nanobodies obtained from sequence analysis and the control antibody H5L5 (Patent CN112714768A, VH sequence is SEQ ID NO: 42, VL sequence is SEQ ID NO: 43) were subcloned into the eukaryotic expression vector pcDNA3.4 together with the IgG4 Fc sequence (SEQ ID NO: 44), and then transfected into Expi293F and cultured for expression for 5 days to prepare bivalent antibodies. The supernatant was collected and purified by Protein A (GenScript, Cat. No. L00695-80).

2.2 Detection of Affinity between CD39 Nanobodies and Human CD39 Protein by Fortebio

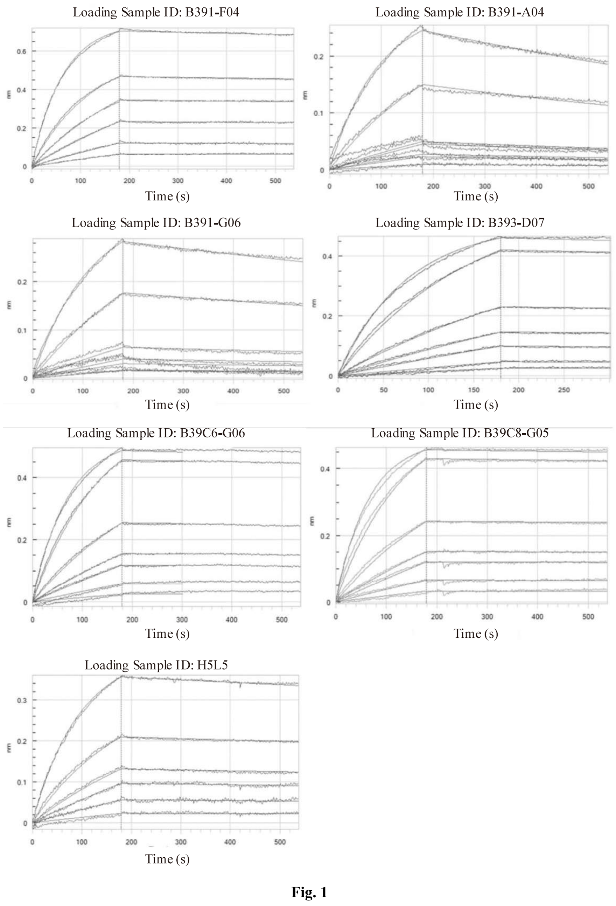

The CD39 antibodies were diluted to 100 nM and added into a 384-well plate. The human CD39-His antigens (ACRO, CD9-H52H4) were diluted to 100 nM and then subjected to a 2-fold serial dilution for a total of 7 gradients, which were then added into the 384-well plate. An AHC probe (Sartorius, Cat. No. 18-5060) was used, with the association time set to 180 seconds, the dissociation time set to 360 seconds, and the baseline set to 60 seconds. Three regeneration cycles were performed, each cycle for 5 seconds, and then the association-dissociation curve was fit with software to calculate the affinity of the antibodies. The results are shown in Fig. 1 and Table 1, indicating that six nanobodies can bind to human CD39 protein with high affinity. Among them, the amino acid sequence of VHH of B391-F04 is SEQ ID NO: 8, the amino acid sequence of VHH of B391-A04 is SEQ ID NO: 16, the amino acid sequence of VHH of B391-G06 is SEQ ID NO: 21, the amino acid sequence of VHH of B393-D07 is SEQ ID NO: 27, the amino acid sequence of VHH of B39C6-G06 is SEQ ID NO: 32, and the amino acid sequence of VHH of B39C8-G05 is SEQ ID NO: 37.

Table 1

| Loading Sample ID | KD (M) | kon(1/Ms) | kdis(1/s) | Full R^2 |
|---|---|---|---|---|
| B391-F04 | 2.55E-10 | 3.15E+05 | 8.05E-05 | 0.9996 |
| B391-A04 | 7.76E-09 | 1.00E+05 | 7.78E-04 | 0.994 |
| B391-G06 | 5.23E-09 | 8.55E+04 | 4.47E-04 | 0.9949 |
| B393-D07 | 1.15E-09 | 1.32E+05 | 1.51E-04 | 0.999 |
| B39C6-G06 | 5.79E-10 | 1.43E+05 | 8.25E-05 | 0.9988 |
| B39C8-G05 | 2.64E-10 | 1.54E+05 | 4.06E-05 | 0.9985 |
| H5L5 | 8.02E-10 | 2.10E+05 | 1.68E-04 | 0.9987 |

2.3 Detection of Binding of CD39 Nanobodies to Cell Surface Human CD39 by Flow Cytometry

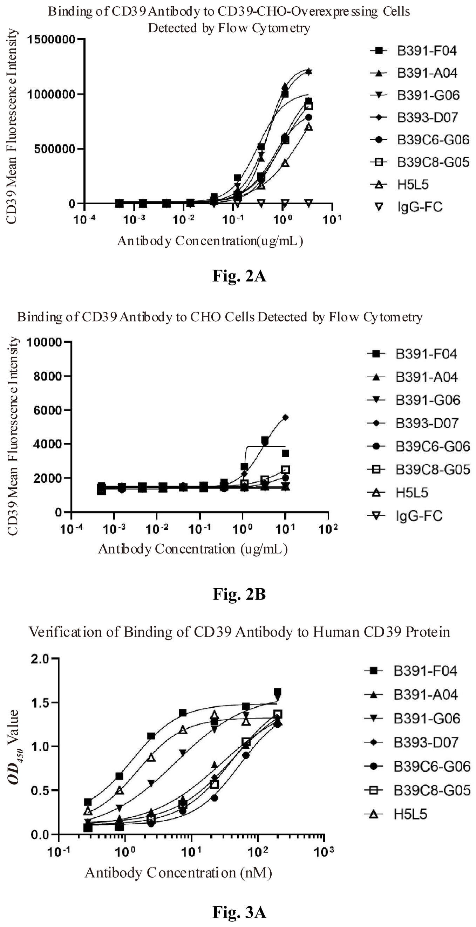

The normally cultured CD39-CHO overexpressing cells and CHO wild-type cells were digested and centrifuged, followed by resuspension in PBS containing 0.1% BSA. A 96-well V-bottom plate was taken, into each well of which were added $4 \times 10^4$ cells, with 30 $\mu$L per well. The CD39 antibodies were formulated at a concentration of 90 $\mu$g/mL (a 60-$\mu$L system) and subjected to a 3-fold serial dilution, with the final dilution containing no antibodies. After dilution, 30 $\mu$L of each antibody dilution was added to the corresponding wells containing cells and mixed thoroughly, followed by incubation at 4 °C for 1 hour. Next, 120 $\mu$L of buffer was added and the mixture was centrifuged at 500 g for 5 minutes. The supernatant was discarded, excess liquid was gently blotted on tissue paper, and the cells were loosened by vortexing. Anti-human IgG-FC dylight650 secondary antibodies (1:200) were added, with 30 $\mu$L per well, and incubated at 4°C for 30 minutes. Then, 150 $\mu$L of buffer was added, and the mixture was centrifuged at 500 g for 5 minutes and washed once more. The supernatant was discarded. The cells were loosened and resuspended with 30 $\mu$L of buffer, and then subjected to detection on the machine. The results are shown in Fig. 2, indicating that six nanobodies can specifically bind to human CD39 membrane protein.

2.4 Detection of Binding of CD39 Antibody to Human, Mouse, Cynomolgus Monkey CD39 Proteins by ELISA

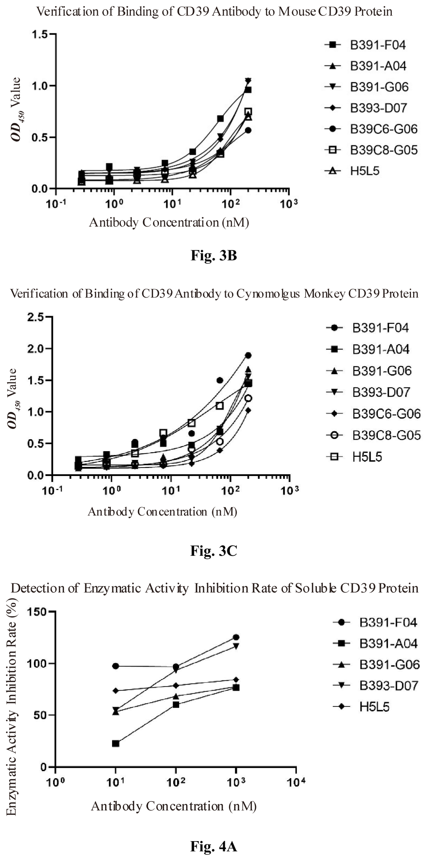

2 $\mu$g/mL of human, mouse, and cynomolgus monkey CD39 proteins from ACRO were coated onto a plate at 30 $\mu$L/well and incubated at 4°C overnight. The next day, the coated plate was washed three times with PBS and blocked with 5% milk at 60 $\mu$L/well at room temperature while shaking at 400 rpm for 2 hours. After three washes with PBST, the CD39 antibodies were diluted with PBS to a highest concentration of 200 nM and then subjected to 3-fold serial dilution, with the last well containing no antibody. The diluted antibodies were added to a 384-well plate at 30 $\mu$L/well and incubated at 400 rpm for 1 hour at room temperature. After three washes with PBST, anti-human IgG-HRP secondary antibodies diluted at 1:10,000 were added at 25 $\mu$L/well and incubated at 400 rpm for 30 minutes at room temperature. After seven washes with PBST, TMB was added for color development (25 $\mu$L/well), and a stop buffer was added at 25 $\mu$L/well after an appropriate time to terminate the reaction, and the absorbance at 450 nm was read. The results are shown in Fig. 3, indicating that all six nanobodies can bind to mouse and cynomolgus monkey CD39 proteins to a certain extent.

2.5 Detection of Enzymatic Activity Inhibition Function of CD39 Antibody at the Protein Level

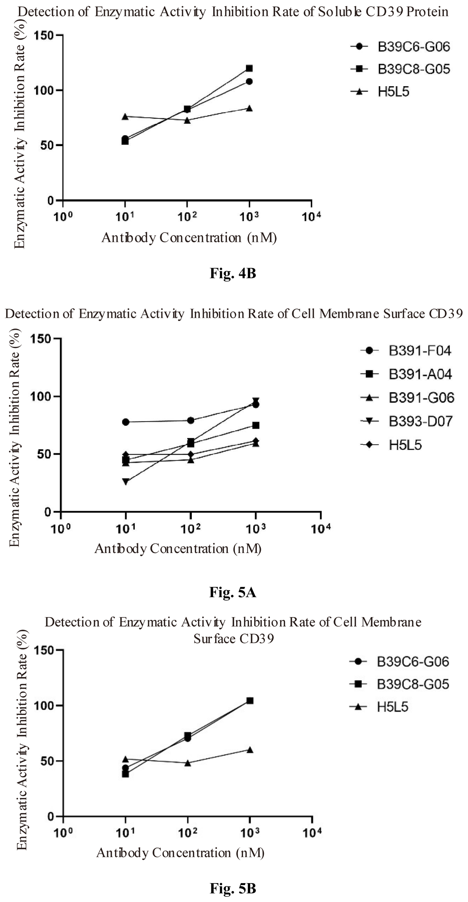

The CD39 antibodies were formulated in Assay Buffer to the highest concentration of 1000 nM and then subjected to 3-fold serial dilution in three gradients. CD39-His protein was prepared at 50 nM, and 5 $\mu$L of CD39 protein was added to each well to a final concentration of 5 nM, followed by the addition of 45 $\mu$L of the diluted CD39 antibody. The mixture was incubated at 37°C for 120 minutes. Subsequently, 50 $\mu$L of 1000 $\mu$M ATP was added to each well and incubated at 37°C for 1 hour. A 25-$\mu$L aliquot of the mixture was transferred to a black 384-well plate, and an equal volume of CellTiter-Glo® Reagent (25 $\mu$L per well) was added. After mixing thoroughly, the plate was shaken on a shaker for 2 minutes and then allowed to stand at room temperature for 10 minutes to stabilize the fluorescence signal, which was then read. The enzymatic activity inhibition rate was calculated according to the formula described below, followed by data plotting. The results are shown in Fig. 4, indicating that all six nanobodies can inhibit the enzymatic activity of CD39 protein to a certain extent.

Calculation formula for inhibition rate of CD39:

$$\frac{(CD39\ protein + Ab + ATP) - (CD39\ protein + ATP)}{(ATP) - (CD39\ protein + ATP)} \times 100\%$$

2.6 Detection of Enzymatic Activity Inhibition Function of CD39 Antibody at the Cellular Level

The SK-MEL-28 cells were digested when they reached about 80% confluence, then seeded at a density of $1.5 \times 10^4$ cells/100 $\mu$L for each well and cultured overnight in a 37°C incubator. The CD39 antibodies were formulated to the highest concentration of 1000 nM and then subjected to 3-fold serial dilution. 50 $\mu$L of the serially diluted antibodies were added at 3 gradients to each well of the overnight-cultured cells and incubated at 37°C for 30 minutes. 50 $\mu$L of 300 $\mu$M ATP was added to each well and incubated at 37°C for 1 hour. A 25-$\mu$L aliquot of the mixture was transferred to a black 384-well plate, and an equal volume of CellTiter-Glo® Reagent (25 $\mu$L per well) was added. After mixing thoroughly, the plate was shaken on a shaker for 2 minutes and then allowed to stand at room temperature for 10 minutes to stabilize the fluorescence signal, which was then read. The enzymatic activity inhibition rate was calculated according to the formula described below, followed by data plotting. The results are shown in Fig. 5, indicating that all six nanobodies can inhibit the enzymatic activity of CD39 membrane protein to a certain extent. Overall, compared with the H5L5 control antibody, the B391-F04 antibody has obvious advantages in the enzymatic activity inhibition function at the cellular and protein levels.

Calculation formula for inhibition rate of CD39:

$$\frac{(CD39\ positive\ cells + Ab + ATP) - (CD39\ positive\ cells + ATP)}{(ATP) - (CD39\ positive\ cells + ATP)} \times 100\%$$

**Example 3 Detection of Promoting Effect of CD39-F04 antibody on Proliferation of PBMC-derived T Cells**

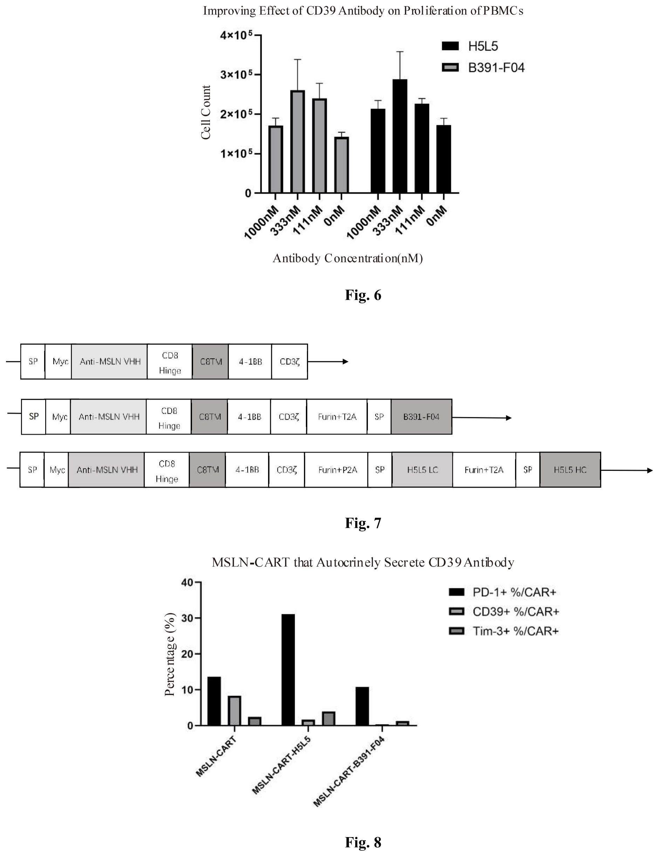

The cryopreserved PBMCs were resuscitated, activated with CD3/CD28 magnetic beads, and plated at $5 \times 10^5$ cells per well. B391-F04 and H5L5 antibodies were diluted to final concentrations of 1000, 333, and 111 nM, respectively. After adding them to the cells, they were incubated at 37°C for 30 minutes. Then, ATP was added to a final concentration of 1000 $\mu$M. After 4 days of co-culture, the cells were counted. The results are shown in Fig. 6, indicating that in the presence of ATP, different concentrations of B391-F04 and H5L5 antibodies both promote T cell proliferation to a certain extent, and the difference between the two is not obvious.

**Example 4 Detection of Expression of Inhibitory Markers on the Surface of MSLN-CART cells that Autocrinely Secrete CD39 Antibody after Stimulation with Tumor Cells**

The MSLN-CART cells (with the structural diagrams shown in Fig. 7) that do not secrete CD39 or autocrinely secrete B391-F04 and H5L5 antibodies (SEQ ID NO: 61 and SEQ ID NO: 62) were co-cultured with SKOV3-MSLN cells overexpressing MSLN for 4 days to detect the expression of the inhibitory markers (PD-1, CD39, Tim-3) on the surface of CAR-T cells. The results are shown in Fig. 8, indicating that compared with the non-secreting control group, in the experimental group, the expression of CD39 on the surface of CART cells is significantly reduced, the effect of B391-F04 is more obvious, and additionally, the expression of PD-1 and Tim-3 exhaustion markers on the CART cells is not upregulated.

**Example 5 Humanization and Function Identification of CD39 antibody**

5.1 Final humanization and affinity determination of B391-F04

Humanization was performed based on the B391-F04 sequence, with the sequence set forth in SEQ ID NO: 41. pcDNA3.4-B391-F04-IgG4 Fc eukaryotic expression plasmids were constructed and transiently transfected into Expi293F to produce antibody proteins, which were then purified by Protein A.

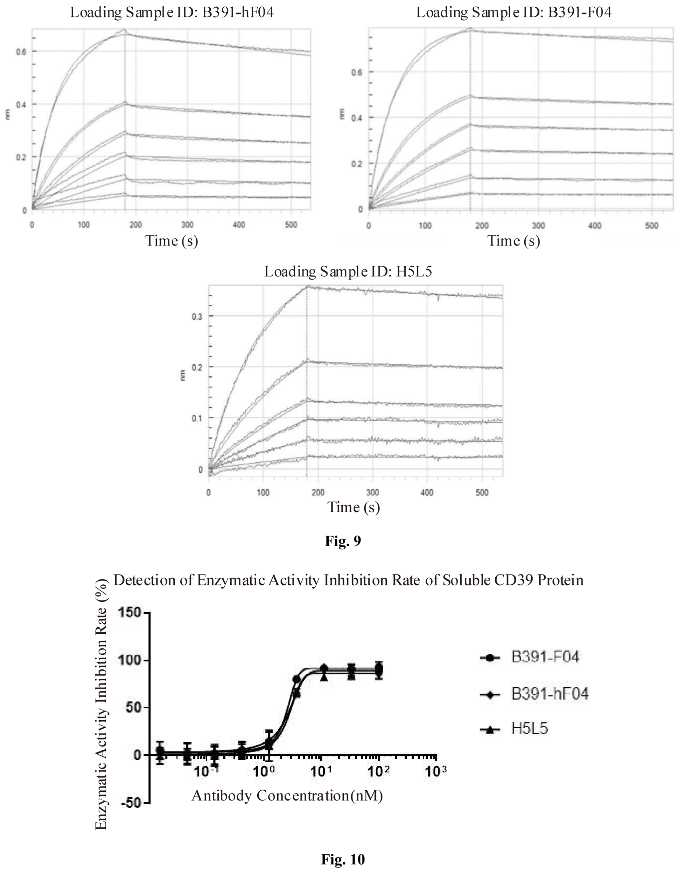

B391-F04, B391-hF04 (VHH sequence is SEQ ID NO: 41), and H5L5 were diluted to 100 nM and the antibodies were added to a 384-well plate. The human CD39-His antigen (ACRO, CD9-H52H4) was subjected to 2-fold dilution for a total of 7 gradients and added to the 384-well plate. An AHC probe (Sartorius, Cat. No. 18-5060) was used, with the association time set to 180 seconds, the dissociation time set to 300 seconds, and the baseline set to 60 seconds. Three regeneration cycles were performed, each cycle for 5 seconds, and then the association-dissociation curve was fit with software to calculate the affinity of the antibodies. The results are shown in Fig. 9 and Table 2, indicating that the affinities of the sequences before and after humanization are not significantly different, and both are better than that of the H5L5 control antibody.

Table 2

| Loading Sample ID | KD (M) | kon(1/Ms) | kdis(1/s) | Full R^2 |
|---|---|---|---|---|
| B391-hF04 | 8.66E-10 | 4.09E+05 | 3.54E-04 | 0.9965 |
| B391-F04 | 3.98E-10 | 4.33E+05 | 1.72E-04 | 0.9989 |
| H5L5 | 1.28E-09 | 3.2E+05 | 4.08E-04 | 0.9943 |

5.2 Detection of Enzymatic Activity Inhibition Function of B391-hF04 Humanized Antibody at the Protein Level

The CD39 antibodies were formulated in Assay buffer to the highest concentration of 100 nM and then subjected to 3-fold serial dilution in nine gradients. CD39-His protein was prepared at 50 nM, and 5 $\mu$L of CD39 protein was added to each well to a final concentration of 5 nM, followed by the addition of 45 $\mu$L of the diluted CD39 antibody. The mixture was incubated at 37°C for 120 minutes. Subsequently, 50 $\mu$L of 1000 $\mu$M ATP was added to each well and incubated at 37°C for 1 hour. A 25-$\mu$L aliquot of the mixture was transferred to a black 384-well plate, and an equal volume of CellTiter-Glo$^{®}$ Reagent (25 $\mu$L per well) was added. After mixing thoroughly, the plate was shaken on a shaker for 2 minutes and then allowed to stand at room temperature for 10 minutes to stabilize the fluorescence signal, which was then read. The enzymatic activity inhibition rate was calculated according to the formula described below, followed by data plotting. The results are shown in Fig. 10, indicating that there are no significant differences among the three compared with the control antibody H5L5.

Calculation formula for inhibition rate of CD39:

$$\frac{(CD39\, protein + Ab + ATP) - (CD39\, protein + ATP)}{(ATP) - (CD39\, protein + ATP)} \times 100\%$$

5.3 Detection of Enzymatic Activity Inhibition Function of B391-hF04 Antibody at the Cellular Level

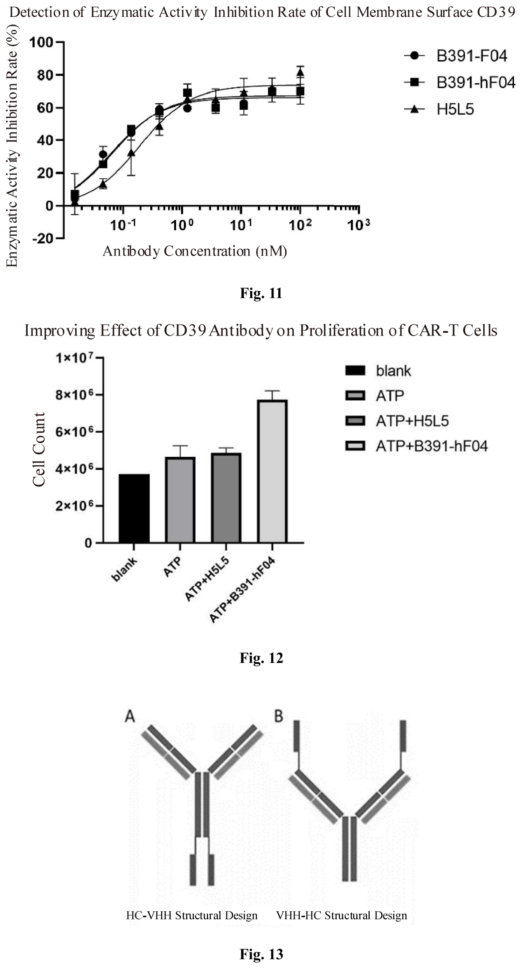

The SK-MEL-28 cells were digested when they reached about 80% confluence, then seeded at a density of $1.5 \times 10^4$ cells/100 $\mu$L for each well and cultured overnight in a 37°C incubator. The CD39 antibodies were formulated to the highest concentration of 100 nM and then subjected to 3-fold serial dilution in nine gradients. 50 $\mu$L of the serially diluted antibodies were added to each well of the overnight-cultured cells and incubated at 37°C for 30 minutes. 50 $\mu$L of 300 $\mu$M ATP was added to each well and incubated at 37°C for 1 hour. A 25-$\mu$L aliquot of the mixture was transferred to a black 384-well plate, and an equal volume of CellTiter-Glo$^{®}$ Reagent (25 $\mu$L per well) was added. After mixing thoroughly, the plate was shaken on a shaker for 2 minutes and then allowed to stand at room temperature for 10 minutes to stabilize the fluorescence signal, which was then read. The enzymatic activity inhibition rate was calculated according to the formula described below, followed by data plotting. The results are shown in Fig. 11. Considering the experimental errors in each group, there are no significant differences among the three compared with the control antibody H5L5.

Calculation formula for inhibition rate of CD39:

$$\frac{(CD39\ positive\ cells + Ab + ATP) - (CD39\ positive\ cells + ATP)}{(ATP) - (CD39\ positive\ cells + ATP)} \times 100\%$$

**Example 6 Detection of Improving Effect of CD39 Humanized Antibody on Proliferation of CAR-T Cells**

The cryopreserved MSLN-CART cells (wherein, the amino acid sequence of CAR was as set forth in SEQ ID NO: 60) were resuscitated, activated with CD3/CD28 magnetic beads, and plated at $5 \times 10^5$ cells per well. B391-hF04 and H5L5 antibodies were diluted to final concentrations of 333 and 111 nM, respectively. After adding them to the cells, they were incubated at 37°C for 30 minutes. Then, ATP was added to a final concentration of 1000 $\mu$M. After 4 days of co-culture, the cells were counted. The results are shown in Fig. 12, indicating that in the presence of a high concentration of ATP, B391-hF04 can significantly improve the proliferation of CAR-T cells compared with the H5L5 control antibody.

**Example 7 Design, Molecular Cloning and Expression Identification of Bispecific Antibody**

The antibody targeting PD-L1 (YN035) is IgG, while the antibody targeting CD39 is a nanobody. On the one hand, PD-L1 is mainly expressed on the surface of tumor cells, and with a small amount expressed on the surface of DC cells. CD39 is mainly expressed on tumor cells and immune cells. If both PD-L1 and CD39 are expressed on the same cell, a PD-L1/CD39 bispecific antibody will theoretically induce cross-linking on the cell surface and therefore can exert a greater inhibitory effect. If only PD-L1 is expressed on the surface of tumor cells, the bispecific antibody can spatially shorten the distance between tumor cells and immune cells and exert a stronger immune function. Therefore, when designing a PD-L1/CD39 bispecific antibody, it is necessary to retain a bivalent binding activity comparable to that of the parental antibody. In addition, considering that CD39 on immune cells is significantly upregulated in the tumor microenvironment, the Fc of IgG4 is used to make it have relatively weak ADCC and CDC activities. The specific design is shown in Fig. 13. The nanobody is respectively linked to the N-terminus and C-terminus of the heavy chain of YN035 (the sequence of HCDR1 is SEQ ID NO: 45, the sequence of HCDR2 is SEQ ID NO: 46, the sequence of HCDR3 is SEQ ID NO: 47, the sequence of VH is SEQ ID NO: 48, the sequence of LCDR1 is SEQ ID NO: 49, the sequence of LCDR2 is SEQ ID NO: 50(GNS), the sequence of LCDR3 is SEQ ID NO: 51, the sequence of VL is SEQ ID NO: 52), wherein the flexible linker is (G4S)n, wherein n=1, 2, 3, 4, and the bispecific antibody is named YN035-n-hF04 and hF04-YN035. By conventional molecular cloning methods, the light and heavy chains of the bispecific antibody sequence were loaded into the eukaryotic expression vector pcDNA3.4. Then, the light and heavy chain expression plasmids were transfected together into Expi293F at a ratio of 2:3. After 5 days of culture in a cell incubator, the supernatant was collected and purified with Protein A (Genscript, Cat. No. L00695-80) and Superdex 200.

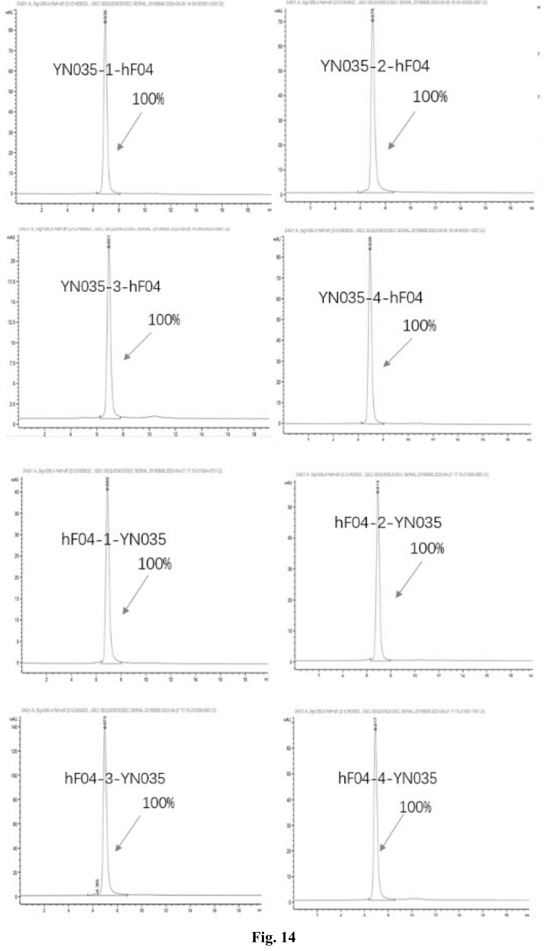

Purity analysis was performed using an SEC chromatography column, with the specific procedures as follows. The high-performance liquid chromatograph (Agilent Technologies 1200 Series) was powered on to first expel the air in the system pipeline with purified water at a flow rate of 2 ml/min. After flushing for 30 minutes, the SET-C SEC-300 (Sepax, Cat. No. 2F36102) was connected to the instrument. The chromatographic column was first flushed with purified water at a flow rate of 0.5 ml/min for 1 hour and then flushed with PBS at a flow rate of 1 ml/min for 1 hour. The sample to be tested was diluted to 1 mg/ml. 100 $\mu$L of the Marker and the diluted sample were added to the sample loading trough tubes, respectively. The loading volume of the marker was set to 6 $\mu$L and the loading volume of the diluted sample to be tested was set to 10 $\mu$L. The flow rate was 1 ml/min, and the procedures run for 20 minutes for sample detection and analysis. The results are shown in Fig. 14, indicating that the constructed bispecific antibodies exhibit good purity. The YN035-3-hF04 bispecific antibody is taken as an example to detect the *in vivo* efficacy.

**Example 8 Verification of *In Vivo* Efficacy of Bispecific Antibody**

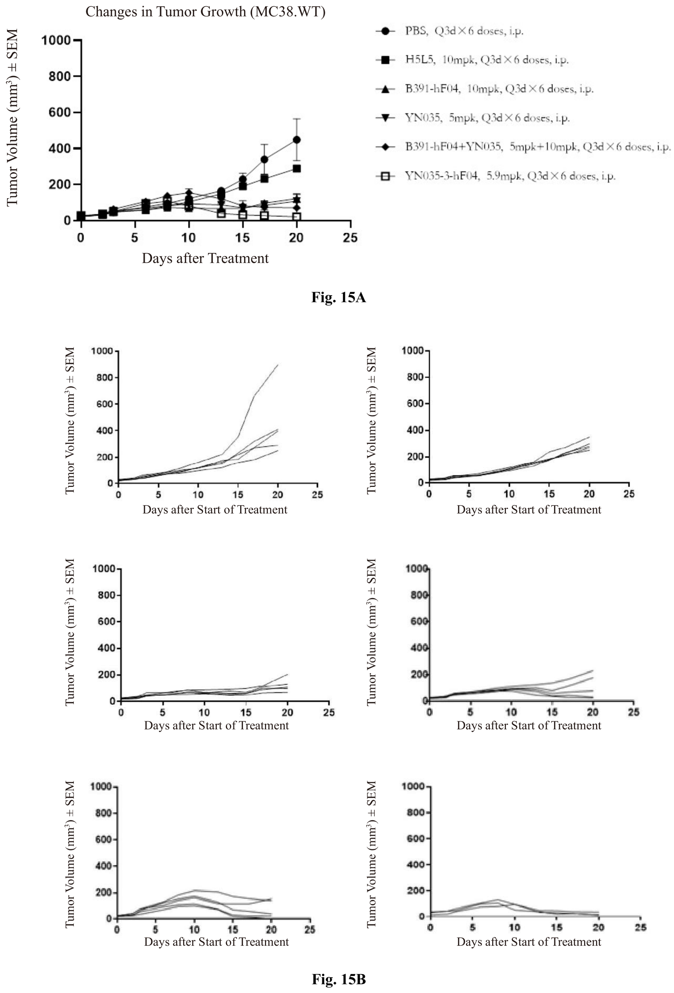

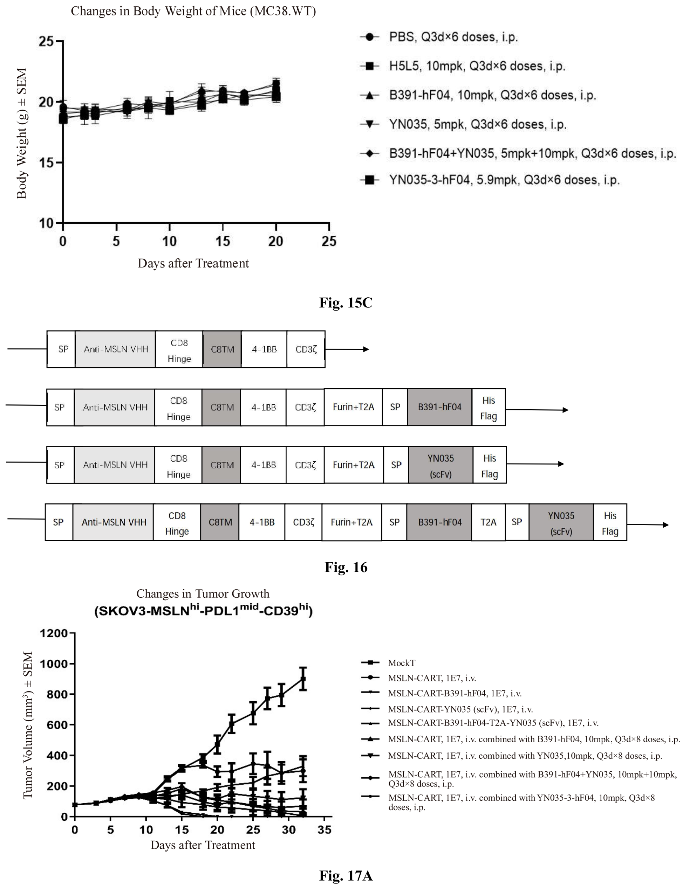

MC38 cells were inoculated subcutaneously on the right flank of C57BL/6-hCD39/hCD73 humanized mice at a concentration of $1 \times 10^5$ cells/0.1 mL. When the tumors grew to approximately 10-20 mm$^2$, the mice were selected by tumor volume and randomly grouped. The administration route for all groups was intraperitoneal injection, with dosing performed once every three days for 6 consecutive times. The experiment ended 5 days after the last administration. The body weight and tumor volume of the mice were measured three times a week during the dosing and observation periods, and the measured values were recorded. At the end of the experiment, the animals were euthanized. The results are shown in Fig. 15. In this animal model, hF04 significantly inhibits the tumor growth compared with the H5L5 control antibody, and the YN035-3-hF04 bispecific antibody has obvious antitumor effects compared with both the control monoclonal antibody and the combined control group.

**Example 9 Verification of *In Vivo* Efficacy of Autocrine Antibody CAR-T and Antibody Combined with Blank CAR-T**

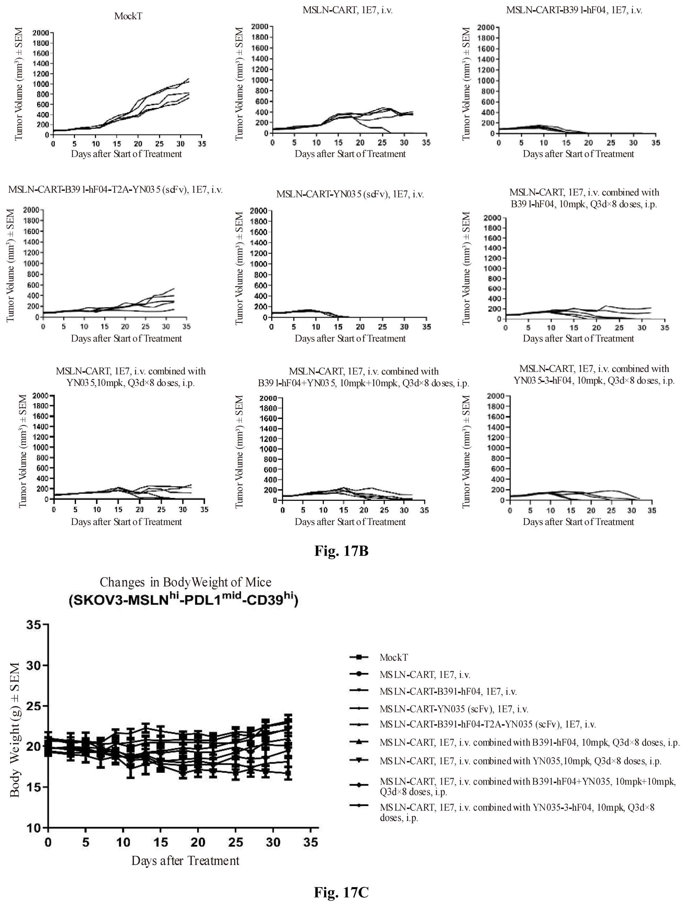

SKOV3-MSLN$^{hi}$-PDL1$^{mid}$-CD39$^{hi}$ ovarian cancer cells were inoculated subcutaneously into the right flank of B-NDG mice at a concentration of $3\times10^6$ cells/0.2 mL. When the tumors grew to approximately 80 mm$^2$, the mice were randomly divided into 9 groups. MockT, MSLN-CART (with the schematic diagram shown in Fig. 16) cells that do not secrete antibodies or autocrinely secrete B391-hF04, YN035 (scFv), B391-hF04+YN035 (scFv) antibodies were injected via the tail vein, with a total of 1 dose administered. At the same time, for the combined treatment group, injections were given via the tail vein once every three days for a total of 8 consecutive doses. Among them, the amino acid sequence of MSLN-CART is as set forth in SEQ ID NO: 60, the amino acid sequence of MSLN-CART-B391-hF04 is as set forth in SEQ ID NO: 63, the amino acid sequence of MSLN-CART-YN035 (scFv) is as set forth in SEQ ID NO: 64, and the amino acid sequence of MSLN-CART-B391-hF04-T2A-YN035 (scFv) is as set forth in SEQ ID NO: 65. During the experiment, if any one or more of the following situations occurred, the experimental animals were euthanized: 1) severe damage to the tumor, with no scab formation within 3 days; 2) abnormal movement or paralysis of the animal; 3) the animal's tumor reached 2000 mm$^3$. The body weight and tumor volume of the mice were measured three times a week during the dosing and observation periods, and the measured values were recorded. At the end of the experiment, the animals were euthanized. The results are shown in Fig. 17. In this animal model, MSLN-CART that autocrinely secretes B391-hF04, YN035 (scFv), B391-hF04+YN035 (scFv) antibodies exhibits an obvious tumor inhibitory effect, and the tumor inhibitory effect of the experimental groups that autocrinely secrete B391-hF04 and YN035 (scFv) antibodies is significantly better than that of the combined application of the antibodies and blank CAR-T.

**Claims**

1. An antibody or an antigen-binding fragment thereof, which can specifically bind to a CD39 protein, having one or more of the following properties:

    (1) capable of binding to CD39 at a KD value of $1\times10^{-8}$ M or below;
    (2) capable of binding to human CD39, mouse CD39, or cynomolgus monkey CD39;
    (3) capable of effectively inhibiting the enzymatic activity of the CD39 protein; and
    (4) capable of inhibiting the growth and/or proliferation of tumor cells.

2. The antibody or the antigen-binding fragment thereof of claim 1, comprising at least one CDR in an antibody heavy chain variable region (VH), wherein said VH comprises an amino acid sequence as set forth in any one of SEQ ID NO: 8, SEQ ID NO: 16, SEQ ID NO: 21, SEQ ID NO: 27, SEQ ID NO: 32, SEQ ID NO: 37, and SEQ ID NO: 41.

3. The antibody or the antigen-binding fragment thereof of any one of claims 1-2, comprising an HCDR3, wherein said HCDR3 comprises an amino acid sequence as set forth in any one of SEQ ID NO: 3, SEQ ID NO: 54, and SEQ ID NO: 55.

4. The antibody or the antigen-binding fragment thereof of any one of claims 1-3, comprising an HCDR3, wherein said HCDR3 comprises an amino acid sequence as set forth in any one of SEQ ID NO: 3, SEQ ID NO: 11, SEQ ID NO: 18, SEQ ID NO: 24, SEQ ID NO: 29, and SEQ ID NO: 33.

5. The antibody or the antigen-binding fragment thereof of any one of claims 1-4, comprising an HCDR2, wherein said HCDR2 comprises an amino acid sequence as set forth in any one of SEQ ID NO: 2, SEQ ID NO: 10, SEQ ID NO: 23, and SEQ ID NO: 28.

6. The antibody or the antigen-binding fragment thereof of any one of claims 1-5, comprising an HCDR1, wherein said HCDR1 comprises an amino acid sequence as set forth in any one of SEQ ID NO: 1, SEQ ID NO: 53, and SEQ ID NO: 22.

7. The antibody or the antigen-binding fragment thereof of any one of claims 1-6, comprising an HCDR1, wherein said HCDR1 comprises an amino acid sequence as set forth in any one of SEQ ID NO: 1, SEQ ID NO: 9, SEQ ID NO: 17, and SEQ ID NO: 22.

8. The antibody or the antigen-binding fragment thereof of any one of claims 1-7, comprising an HCDR1, an HCDR2, and an HCDR3, wherein said HCDR1, HCDR2, and HCDR3 comprise amino acid sequences selected from a group consisting of:

(1) said HCDR1 comprises an amino acid sequence as set forth in SEQ ID NO: 1, said HCDR2 comprises an amino acid sequence as set forth in SEQ ID NO: 2, and said HCDR3 comprises an amino acid sequence as set forth in SEQ ID NO: 3;
(2) said HCDR1 comprises an amino acid sequence as set forth in SEQ ID NO: 9, said HCDR2 comprises an amino acid sequence as set forth in SEQ ID NO: 10, and said HCDR3 comprises an amino acid sequence as set forth in SEQ ID NO: 11;
(3) said HCDR1 comprises an amino acid sequence as set forth in SEQ ID NO: 17, said HCDR2 comprises an amino acid sequence as set forth in SEQ ID NO: 10, and said HCDR3 comprises an amino acid sequence as set forth in SEQ ID NO: 18;
(4) said HCDR1 comprises an amino acid sequence as set forth in SEQ ID NO: 22, said HCDR2 comprises an amino acid sequence as set forth in SEQ ID NO: 23, and said HCDR3 comprises an amino acid sequence as set forth in SEQ ID NO: 24;
(5) said HCDR1 comprises an amino acid sequence as set forth in SEQ ID NO: 22, said HCDR2 comprises an amino acid sequence as set forth in SEQ ID NO: 28, and said HCDR3 comprises an amino acid sequence as set forth in SEQ ID NO: 29; or
(6) said HCDR1 comprises an amino acid sequence as set forth in SEQ ID NO: 22, said HCDR2 comprises an amino acid sequence as set forth in SEQ ID NO: 28, and said HCDR3 comprises an amino acid sequence as set forth in SEQ ID NO: 33.

9. The antibody or the antigen-binding fragment thereof of any one of claims 1-8, comprising an H-FR1, wherein a C-terminus of said H-FR1 is linked to an N-terminus of said HCDR1 directly or indirectly, and said H-FR1 comprises an amino acid sequence as set forth in SEQ ID NO: 56.

10. The antibody or the antigen-binding fragment thereof of claim 9, wherein said H-FR1 comprises an amino acid sequence as set forth in any one of SEQ ID NO: 4, SEQ ID NO: 12, SEQ ID NO: 19, SEQ ID NO: 34, and SEQ ID NO: 38.

11. The antibody or the antigen-binding fragment thereof of any one of claims 1-10, comprising an H-FR2, wherein said H-FR2 is located between said HCDR1 and said HCDR2, and said H-FR2 comprises an amino acid sequence as set forth in SEQ ID NO: 57.

12. The antibody or the antigen-binding fragment thereof of any one of claims 1-11, wherein said H-FR2 comprises an amino acid sequence as set forth in any one of SEQ ID NO: 5, SEQ ID NO: 13, SEQ ID NO: 20, SEQ ID NO: 25, SEQ ID NO: 30, and SEQ ID NO: 35.

13. The antibody or the antigen-binding fragment thereof of any one of claims 1-12, comprising an H-FR3, wherein said H-FR3 is located between said HCDR2 and said HCDR3, and said H-FR3 comprises an amino acid sequence as set forth in SEQ ID NO: 58.

14. The antibody or the antigen-binding fragment thereof of claim 13, wherein said H-FR3 comprises an amino acid sequence as set forth in any one of SEQ ID NO: 6, SEQ ID NO: 14, SEQ ID NO: 26, SEQ ID NO: 31, SEQ ID NO: 36, and SEQ ID NO: 40.

15. The antibody or the antigen-binding fragment thereof of any one of claims 1-14, comprising an H-FR4, wherein an N-terminus of said H-FR4 is linked to a C-terminus of said HCDR3, and said H-FR4 comprises an amino acid sequence as set forth in SEQ ID NO: 59.

16. The antibody or the antigen-binding fragment thereof of claim 15, wherein said H-FR4 comprises an amino acid sequence as set forth in any one of SEQ ID NO: 7, SEQ ID NO: 15, and SEQ ID NO: 39.

17. The antibody or the antigen-binding fragment thereof of any one of claims 1-16, comprising a VH, wherein said VH comprises an amino acid sequence as set forth in any one of SEQ ID NO: 8, SEQ ID NO: 16, SEQ ID NO: 21, SEQ ID NO: 27, SEQ ID NO: 32, SEQ ID NO: 37, and SEQ ID NO: 41.

18. The antibody or the antigen-binding fragment thereof of any one of claims 1-17, being a VHH.

19. The antibody or the antigen-binding fragment thereof of claim 18, wherein said VHH comprises an amino acid sequence as set forth in any one of SEQ ID NO: 8, SEQ ID NO: 16, SEQ ID NO: 21, SEQ ID NO: 27, SEQ ID NO: 32, SEQ ID NO: 37, and SEQ ID NO: 41.

20. The antibody or the antigen-binding fragment thereof of any one of claims 1-19, comprising an antibody heavy chain constant region.

21. The antibody or the antigen-binding fragment thereof of claim 20, wherein said antibody heavy chain constant region is derived from a human IgG constant region.

22. The antibody or the antigen-binding fragment thereof of any one of claims 20-21, wherein said antibody heavy chain constant region is derived from a human IgG4 constant region.

23. The antibody or the antigen-binding fragment thereof of any one of claims 20-22, wherein said antibody heavy chain constant region comprises an amino acid sequence as set forth in SEQ ID NO: 44.

24. The antibody or the antigen-binding fragment thereof of any one of claims 1-23, wherein said antigen-binding fragment comprises Fab, Fab', an Fv fragment, F(ab')$_2$, F(ab)$_2$, scFv, di-scFv and/or dAb.

25. The antibody or the antigen-binding fragment thereof of any one of claims 1-24, wherein said antibody is selected from one or more of a group consisting of: a monoclonal antibody, a chimeric antibody, a humanized antibody, and a fully human antibody.

26. A bispecific antibody or an antigen-binding fragment thereof, comprising a first binding domain that specifically binds to a CD39 protein, wherein said first binding domain comprises the antibody or the antigen-binding fragment thereof of any one of claims 1-25.

27. The bispecific antibody or the antigen-binding fragment thereof of claim 26, wherein said first binding domain that specifically binds to CD39 is a VHH that specifically binds to CD39.

28. The bispecific antibody or the antigen-binding fragment thereof of any one of claims 26-27, comprising a second binding domain that specifically binds to a PD-L1 protein.

29. The bispecific antibody or the antigen-binding fragment thereof of claim 28, wherein said second binding domain that specifically binds to the PD-L1 protein comprises a PD-L1 antibody or an antigen-binding fragment thereof.

30. The bispecific antibody or the antigen-binding fragment thereof of claim 29, wherein said PD-L1 antibody or the antigen-binding fragment thereof comprises an HCDR1, an HCDR2, and an HCDR3, and said HCDR1 comprises an amino acid sequence as set forth in SEQ ID NO: 45, said HCDR2 comprises an amino acid sequence as set forth in SEQ ID NO: 46, and said HCDR3 comprises an amino acid sequence as set forth in SEQ ID NO: 47.

31. The bispecific antibody or the antigen-binding fragment thereof of any one of claims 29-30, wherein said PD-L1 antibody or the antigen-binding fragment thereof comprises a VH, and said VH comprises an amino acid sequence as set forth in SEQ ID NO: 48.

32. The bispecific antibody or the antigen-binding fragment thereof of any one of claims 29-31, wherein said PD-L1 antibody or the antigen-binding fragment thereof comprises an LCDR1, an LCDR2, and an LCDR3, and said LCDR1 comprises an amino acid sequence as set forth in SEQ ID NO: 49, said LCDR2 comprises an amino acid sequence as set forth in SEQ ID NO: 50(GNS), and said LCDR3 comprises an amino acid sequence as set forth in SEQ ID NO: 51.

33. The bispecific antibody or the antigen-binding fragment thereof of any one of claims 29-32, wherein said PD-L1 antibody or the antigen-binding fragment thereof comprises a VL, and said VL comprises an amino acid sequence as set forth in SEQ ID NO: 52.

34. The bispecific antibody or the antigen-binding fragment thereof of any one of claims 29-33, wherein said PD-L1 antibody or the antigen-binding fragment thereof comprises an antibody heavy chain constant region.

35. The bispecific antibody or the antigen-binding fragment thereof of claim 34, wherein said antibody heavy chain constant region is derived from a human IgG constant region.

36. The bispecific antibody or the antigen-binding fragment thereof of any one of claims 29-35, wherein said PD-L1 antibody or the antigen-binding fragment thereof comprises an antibody light chain constant region.

37. The bispecific antibody or the antigen-binding fragment thereof of any one of claims 29-36, wherein a C-terminus of said VHH that specifically binds to CD39 is linked to an N-terminus of said PD-L1 antibody or the antigen-binding fragment thereof directly or indirectly.

38. The bispecific antibody or the antigen-binding fragment thereof of any one of claims 29-36, wherein an N-terminus of said VHH that specifically binds to CD39 is linked to a C-terminus of said PD-L1 antibody or the antigen-binding fragment thereof directly or indirectly.

39. The bispecific antibody or the antigen-binding fragment thereof of claim 38, wherein said indirect linkage comprises linkage through a linker.

40. The bispecific antibody or the antigen-binding fragment thereof of claim 39, wherein said linker comprises an amino acid sequence as set forth in (G4S)n, wherein n is a positive integer from 1 to 10.

41. The bispecific antibody or the antigen-binding fragment thereof of any one of claims 29-40, comprising a first polypeptide chain and a second polypeptide chain, wherein said first polypeptide chain comprises a heavy chain variable region of said PD-L1 antibody or an antigen-binding fragment thereof, a heavy chain constant region of said PD-L1 antibody or an antigen-binding fragment thereof, and said VHH that specifically binds to CD39, and said second polypeptide chain comprises a light chain variable region and a light chain constant region of said PD-L1 antibody or the antigen-binding fragment thereof.

42. The bispecific antibody or the antigen-binding fragment thereof of any one of claims 29-41, comprising a first polypeptide chain and a second polypeptide chain, wherein said first polypeptide chain comprises the heavy chain variable region of said PD-L1 antibody or the antigen-binding fragment thereof, the heavy chain constant region of said PD-L1 antibody or the antigen-binding fragment thereof, a linker, and said VHH that specifically binds to CD39, and said second polypeptide chain comprises the light chain variable region and the light chain constant region of said PD-L1 antibody or the antigen-binding fragment thereof.

43. The bispecific antibody or the antigen-binding fragment thereof of any one of claims 29-42, comprising a first polypeptide chain and a second polypeptide chain, wherein said first polypeptide chain comprises, sequentially from N-terminus to C-terminus: the heavy chain variable region of said PD-L1 antibody or the antigen-binding fragment thereof, the heavy chain constant region of said PD-L1 antibody or the antigen-binding fragment thereof, a linker, and said VHH that specifically binds to CD39, wherein said second polypeptide chain comprises the light chain variable region and the light chain constant region of said PD-L1 antibody or the antigen-binding fragment thereof.

44. The bispecific antibody or the antigen-binding fragment thereof of any one of claims 29-42, comprising a first polypeptide chain and a second polypeptide chain, wherein said first polypeptide chain comprises, sequentially from N-terminus to C-terminus: said VHH that specifically binds to CD39, a linker, the heavy chain variable region of said PD-L1 antibody or the antigen-binding fragment thereof, and the heavy chain constant region of said PD-L1 antibody or the antigen-binding fragment thereof, wherein said second polypeptide chain comprises the light chain variable region and the light chain constant region of said PD-L1 antibody or the antigen-binding fragment thereof.

45. A polypeptide, comprising the antibody or the antigen-binding fragment thereof of any one of claims 1-25, or the bispecific antibody or the antigen-binding fragment thereof of any one of claims 26-44.

46. One or more isolated nucleic acid molecules, encoding the antibody or the antigen-binding fragment thereof of any one of claims 1-25, or the bispecific antibody or the antigen-binding fragment thereof of any one of claims 26-44.

47. The nucleic acid molecule of claim 46, further comprising nucleic acid molecule encoding other proteins.

48. The nucleic acid molecule of claim 47, wherein said other proteins comprise chimeric antigen receptors (CARs).

49. A vector, comprising the nucleic acid molecule of any one of claims 46-48.

50. The vector of claim 49, wherein said nucleic acid molecule encoding the other proteins and said nucleic acid molecule encoding the antibody or the antigen-binding fragment thereof are located within the same vector.

51. The vector of claim 49, wherein said nucleic acid molecule encoding the other proteins and said nucleic acid molecule encoding the antibody or the antigen-binding fragment thereof are located within different vectors.

52. A cell, comprising the nucleic acid molecule of any one of claims 46-48, or the vector of any one of claims 49-51.

53. The cell of claim 52, comprising an immune cell.

54. The cell of claim 53, wherein said immune cell comprises a T cell, an NK cell, and/or a macrophage.

55. The cell of any one of claims 5-54, further comprising and/or expressing other proteins.

56. The cell of claim 55, wherein said other proteins comprise chimeric antigen receptors (CARs).

57. A drug combination, comprising the following components:

   (1) the antibody or the antigen-binding fragment thereof of any one of claims 1-25, or the bispecific antibody or the antigen-binding fragment thereof of any one of claims 26-44;
   (2) a cell.

58. The drug combination of claim 57, wherein said cell comprises a modified cell or an unmodified cell.

59. The drug combination of any one of claims 57-58, wherein said modified cell comprises and/or expresses a CAR.

60. The drug combination of any one of claims 57-59, wherein said cell comprises an immune cell.

61. The drug combination of any one of claims 57-60, wherein said cell comprises a T cell, an NK cell, and/or a macrophage.

62. A method for preparing the antibody or the antigen-binding fragment thereof of any one of claims 1-25 or the bispecific antibody or the antigen-binding fragment thereof of any one of claims 26-44, comprising culturing the cell of any one of claims 52-56 under conditions enabling the expression of the antibody or the antigen-binding fragment thereof of any one of claims 1-25 or the bispecific antibody or the antigen-binding fragment thereof of any one of claims 26-44.

63. A pharmaceutical composition, comprising the antibody or the antigen-binding fragment thereof of any one of claims 1-25, the bispecific antibody or the antigen-binding fragment thereof of any one of claims 26-44, the nucleic acid molecule of any one of claims 46-48, the vector of any one of claims 49-51, the cell of any one of claims 52-56, and/or the drug combination of any one of claims 57-61, and optionally a pharmaceutically acceptable carrier.

64. Use of the antibody or the antigen-binding fragment thereof of any one of claims 1-25, the bispecific antibody or the antigen-binding fragment thereof of any one of claims 26-44, the nucleic acid molecule of any one of claims 46-48, the vector of any one of claims 49-51, the cell of any one of claims 52-56, the drug combination of any one of claims 57-61, and/or the pharmaceutical composition of claim 63 in the preparation of a drug for preventing and/or treating a disease and/or a disorder.

65. The use of claim 64, wherein said disease and/or disorder comprises a tumor.

66. The use of claim 65, wherein said tumor comprises a solid tumor and/or a hematological tumor.

67. The use of any one of claims 65-66, wherein said tumor comprises a tumor affected by CD39 activity.

68. A method for preventing and/or treating a disease and/or a disorder, comprising administering to a subject in need thereof the antibody or the antigen-binding fragment thereof of any one of claims 1-25, the bispecific antibody or the antigen-binding fragment thereof of any one of claims 26-44, the nucleic acid molecule of any one of claims 46-48, the vector of any one of claims 49-51, the cell of any one of claims 52-56, the drug combination of any one of claims 57-61, and/or the pharmaceutical composition of claim 63.

69. The method of claim 68, wherein said disease and/or disorder comprises a tumor.

70. The method of claim 69, wherein said tumor comprises a solid tumor and/or a hematological tumor.

71. The method of any one of claims 69-70, wherein said tumor comprises a tumor affected by CD39 activity.

**Fig. 1**

Binding of CD39 Antibody to CD39-CHO-Overexpressing Cells
Detected by Flow Cytometry

**Fig. 2A**

Binding of CD39 Antibody to CHO Cells Detected by Flow Cytometry

**Fig. 2B**

Verification of Binding of CD39 Antibody to Human CD39 Protein

**Fig. 3A**

Verification of Binding of CD39 Antibody to Mouse CD39 Protein

**Fig. 3B**

Verification of Binding of CD39 Antibody to Cynomolgus Monkey CD39 Protein

**Fig. 3C**

Detection of Enzymatic Activity Inhibition Rate of Soluble CD39 Protein

**Fig. 4A**

Detection of Enzymatic Activity Inhibition Rate of Soluble CD39 Protein

**Fig. 4B**

Detection of Enzymatic Activity Inhibition Rate of Cell Membrane Surface CD39

**Fig. 5A**

Detection of Enzymatic Activity Inhibition Rate of Cell Membrane Surface CD39

**Fig. 5B**

Improving Effect of CD39 Antibody on Proliferation of PBMCs

**Fig. 6**

**Fig. 7**

MSLN-CART that Autocrinely Secrete CD39 Antibody

**Fig. 8**

Loading Sample ID: B391-hF04

Loading Sample ID: B391-F04

Time (s)

Time (s)

Loading Sample ID: H5L5

Time (s)

**Fig. 9**

Detection of Enzymatic Activity Inhibition Rate of Soluble CD39 Protein

Enzymatic Activity Inhibition Rate (%)

Antibody Concentration(nM)

B391-F04

B391-hF04

H5L5

**Fig. 10**

Detection of Enzymatic Activity Inhibition Rate of Cell Membrane Surface CD39

**Fig. 11**

Improving Effect of CD39 Antibody on Proliferation of CAR-T Cells

**Fig. 12**

HC-VHH Structural Design        VHH-HC Structural Design

**Fig. 13**

**Fig. 14**

Changes in Tumor Growth (MC38.WT)

**Fig. 15A**

**Fig. 15B**

Changes in Body Weight of Mice (MC38.WT)

- PBS, Q3d×6 doses, i.p.
- H5L5, 10mpk, Q3d×6 doses, i.p.
- B391-hF04, 10mpk, Q3d×6 doses, i.p.
- YN035, 5mpk, Q3d×6 doses, i.p.
- B391-hF04+YN035, 5mpk+10mpk, Q3d×6 doses, i.p.
- YN035-3-hF04, 5.9mpk, Q3d×6 doses, i.p.

**Fig. 15C**

**Fig. 16**

Changes in Tumor Growth
**(SKOV3-MSLN$^{hi}$-PDL1$^{mid}$-CD39$^{hi}$)**

- MockT
- MSLN-CART, 1E7, i.v.
- MSLN-CART-B391-hF04, 1E7, i.v.
- MSLN-CART-YN035 (scFv), 1E7, i.v.
- MSLN-CART-B391-hF04-T2A-YN035 (scFv), 1E7, i.v.
- MSLN-CART, 1E7, i.v. combined with B391-hF04, 10mpk, Q3d×8 doses, i.p.
- MSLN-CART, 1E7, i.v. combined with YN035,10mpk, Q3d×8 doses, i.p.
- MSLN-CART, 1E7, i.v. combined with B391-hF04+YN035, 10mpk+10mpk, Q3d×8 doses, i.p.
- MSLN-CART, 1E7, i.v. combined with YN035-3-hF04, 10mpk, Q3d×8 doses, i.p.

**Fig. 17A**

**Fig. 17B**

**Fig. 17C**

## INTERNATIONAL SEARCH REPORT

| International application No. |
| --- |
| **PCT/CN2024/096977** |

**A.     CLASSIFICATION OF SUBJECT MATTER**

C07K16/28(2006.01)i;   C12N15/13(2006.01)i;   A61K39/395(2006.01)i;   A61P35/00(2006.01)i

According to International Patent Classification (IPC) or to both national classification and IPC

**B.     FIELDS SEARCHED**

Minimum documentation searched (classification system followed by classification symbols)

IPC:C07K,C12N,A61K,A61P

Documentation searched other than minimum documentation to the extent that such documents are included in the fields searched

Electronic data base consulted during the international search (name of data base and, where practicable, search terms used)

CNTXT, DWPI, ENTXT, ENTXTC, VEN, WPABS, WPABSC, PUBMED, ISI WEB OF SCIENCE, GenBank, STN: 抗体, antibody, VH, VL, CDR, FR, CH2, CH3, IgG, SEQ ID NOs: 1-52, 原启生物, CD39, PD L1, PD-L1, VHH, 纳米抗体, 肿瘤, 癌症, tumor, cancer

**C.     DOCUMENTS CONSIDERED TO BE RELEVANT**

| Category* | Citation of document, with indication, where appropriate, of the relevant passages | Relevant to claim No. |
| --- | --- | --- |
| X | US 2021388105 A1 (ELPISCIENCE (SUZHOU) BIOPHARMA, LTD. et al.) 16 December 2021 (2021-12-16)<br>description, paragraphs 3-71, and embodiment 1 | 1, 18, 20-29, 34-71 |
| Y | US 2021388105 A1 (ELPISCIENCE (SUZHOU) BIOPHARMA, LTD. et al.) 16 December 2021 (2021-12-16)<br>description, paragraphs 3-71, and embodiment 1 | 9-16, 30-33 |
| Y | CN 112409483 A (ZHEJIANG DOER BIOLOGICS CORPORATION) 26 February 2021 (2021-02-26)<br>description, paragraphs 6-11 | 9-16 |
| Y | CN 113087799 A (ORICELL THERAPEUTICS (SHANGHAI) CO., LTD.) 09 July 2021 (2021-07-09)<br>description, paragraphs 6-11 | 30-33 |
| A | CN 116648462 A (NANJING ZAIMING PHARMACEUTICAL CO., LTD.) 25 August 2023 (2023-08-25)<br>claims 1-17 | 1-71 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **04 September 2024** | **10 September 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/096977** |

### C.   DOCUMENTS CONSIDERED TO BE RELEVANT

| Category* | Citation of document, with indication, where appropriate, of the relevant passages | Relevant to claim No. |
| --- | --- | --- |
| A | CN 111699198 A (NANJING LEGEND BIOTECH CO., LTD.) 22 September 2020 (2020-09-22)<br>      claims 1-70 | 1-71 |
| A | 李慧娟 等 (LI, Huijuan et al.). "CD39在肿瘤免疫中的研究进展 (Non-official translation: Research Progress of CD39 in Tumor Immunity)"<br>东南国防医药 (Military Medical Journal of Southeast China),<br>Vol. 20, No. (1), 31 January 2018 (2018-01-31),<br>      pp. 45-49 | 1-71 |
| A | MICHAUD, M. et al. "Subversion of the Chemotherapy-induced Anticancer Immune Response by the Ecto-ATPase CD39"<br>OncoImmunology, Vol. 1, No. (3), 30 June 2012 (2012-06-30),<br>      pp. 392-394 | 1-71 |

Form PCT/ISA/210 (second sheet) (July 2022)

**INTERNATIONAL SEARCH REPORT**

International application No.

**PCT/CN2024/096977**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/CN2024/096977**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **68-71**
   because they relate to subject matter not required to be searched by this Authority, namely:

   Claims 68-71 relate to a method for treatment of a disease, which falls within disease treatment methods as defined in PCT Rule 39.1(iv). However, a search is still carried out on the basis of the pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/CN2024/096977** |

| Patent document cited in search report | | | Publication date (day/month/year) | Patent family member(s) | | | Publication date (day/month/year) |
|---|---|---|---|---|---|---|---|
| US | 2021388105 | A1 | 16 December 2021 | TW | 202124453 | A | 01 July 2021 |
| | | | | EP | 3820907 | A1 | 19 May 2021 |
| | | | | EP | 3820907 | A4 | 04 May 2022 |
| | | | | WO | 2021037037 | A1 | 04 March 2021 |
| | | | | KR | 20220050971 | A | 25 April 2022 |
| | | | | JP | 2022545585 | A | 28 October 2022 |
| | | | | AU | 2020286284 | A1 | 18 March 2021 |
| | | | | AU | 2020286284 | B2 | 09 November 2023 |
| CN | 112409483 | A | 26 February 2021 | | None | | |
| CN | 113087799 | A | 09 July 2021 | SG | 11202009791 | VA | 27 November 2020 |
| | | | | EP | 3778635 | A1 | 17 February 2021 |
| | | | | EP | 3778635 | A4 | 26 January 2022 |
| | | | | CO | 2020013833 | A2 | 21 December 2020 |
| | | | | IL | 277893 | A | 30 November 2020 |
| | | | | KR | 20200140882 | A | 16 December 2020 |
| | | | | WO | 2019196309 | A1 | 17 October 2019 |
| | | | | MX | 2020010653 | A | 28 October 2020 |
| | | | | AU | 2018418224 | A1 | 26 November 2020 |
| | | | | BR | 112020020637 | A2 | 23 March 2021 |
| | | | | PH | 12020551661 | A1 | 07 June 2021 |
| | | | | CL | 2020002600 | A1 | 29 January 2021 |
| | | | | US | 2021380698 | A1 | 09 December 2021 |
| | | | | US | 11993655 | B2 | 28 May 2024 |
| | | | | EA | 202092420 | A1 | 28 January 2021 |
| | | | | JP | 2021530430 | A | 11 November 2021 |
| | | | | JP | 7360720 | B2 | 13 October 2023 |
| | | | | IL | 297847 | A | 01 January 2023 |
| | | | | IL | 297847 | B1 | 01 October 2023 |
| | | | | IL | 297847 | B2 | 01 February 2024 |
| | | | | JP | 2023179558 | A | 19 December 2023 |
| | | | | ZA | 202006255 | B | 30 March 2022 |
| | | | | CA | 3095498 | A1 | 17 October 2019 |
| CN | 116648462 | A | 25 August 2023 | WO | 2022135536 | A1 | 30 June 2022 |
| CN | 111699198 | A | 22 September 2020 | US | 2024124580 | A1 | 18 April 2024 |
| | | | | KR | 20200104333 | A | 03 September 2020 |
| | | | | JP | 2023160982 | A | 02 November 2023 |
| | | | | CA | 3082280 | A1 | 04 July 2019 |
| | | | | AU | 2018396970 | A1 | 13 August 2020 |
| | | | | IL | 275575 | A | 31 August 2020 |
| | | | | JP | 2021508469 | A | 11 March 2021 |
| | | | | JP | 7369127 | B2 | 25 October 2023 |
| | | | | TW | 201930358 | A | 01 August 2019 |
| | | | | US | 2021054071 | A1 | 25 February 2021 |
| | | | | US | 11905327 | B2 | 20 February 2024 |
| | | | | EP | 3732202 | A1 | 04 November 2020 |
| | | | | EP | 3732202 | A4 | 15 June 2022 |
| | | | | SG | 11202004158 | QA | 29 June 2020 |
| | | | | WO | 2019129221 | A1 | 04 July 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 112714768 A

**Non-patent literature cited in the description**

- **CLACKSON et al.** *Nature*, 1991, vol. 352, 624-628
- **MARKS et al.** *Mol. Biol.*, 1991, vol. 222, 581-597
- **JONES et al.** *Nature*, 1986, vol. 321, 522
- **VERHOEYEN et al.** *Science*, vol. 239, 1534-1536
- **RIECHMANN et al.** *Nature*, 1988, vol. 332, 323-337
- **TEMPEST et al.** *Bio/Technol*, 1991, vol. 9, 266-271
- **QUEEN et al.** *Proc Natl Acad Sci USA*, 1989, vol. 86, 10029-10033
- **; CO et al.** *Proc Natl Acad Sci USA*, 1991, vol. 88, 2869-2873
- **CO et al.** *J Immunol*, 1992, vol. 148, 1149-1154
- Derivation of therapeutically active humanized and veneered anti-CD18 antibodies. **MARK et al.** Cellular adhesion: molecular definition to therapeutic potential. Plenum Press, 1994, 291-312
- **FANGER, MICHAEL W.** ; **PAUL M. GUYRE**. Bispecific antibodies for targeted cellular cytotoxicity. *Trends in biotechnology*, 1991, 375-380
- **FAN, GAOWEI et al.** Bispecific antibodies and their applications. *Journal of hematology & oncology*, 2015, 130
- **W. R. PEARSON** ; **D. J. LIPMAN**. Improved tools for biological sequence comparison. *Proc. Natl. Acad. Sci.*, 1988, vol. 85, 2444-2448
- **D. J. LIPMAN** ; **W. R. PEARSON**. Rapid and Sensitive Protein Similarity Searches. *Science*, 1989, vol. 227, 1435-1441
- **S. ALTSCHUL** ; **W. GISH** ; **W. MILLER** ; **E. W. MYERS** ; **D. LIPMAN**. Basic Local Alignment Search Tool. *Journal of Molecular Biology*, 1990, vol. 215, 403-410
- **SAMBROOK et al.** Molecular Cloning, A Laboratory Manual. Cold Spring Harbor Laboratory Press, 1989
- **AUSUBE et al.** Current Protocols in Molecular Biology. Greene Publishing and Wiley-Interscience, 1993